(19) Europäisches Patentamt
European Patent Office
Office européen des brevets

(11) Publication number: **0 023 923**
**B1**

(12) **EUROPEAN PATENT SPECIFICATION**

(45) Date of publication of patent specification: **14.08.85**

(51) Int. Cl.[4]: **C 07 C 45/50**, C 07 C 27/20, C 07 F 7/08, C 07 F 9/50, B 01 J 31/02, B 01 J 31/12

(21) Application number: **80900539.0**

(22) Date of filing: **12.02.80**

(86) International application number: **PCT/US80/00212**

(87) International publication number: **WO 80/01689 21.08.80 Gazette 80/19**

# (54) COMPOUNDS AND CATALYSTS CONTAINING PHOSPHORUS, ARSENIC OR NITROGEN AND THEIR USE IN HYDROCARBON CONVERSION PROCESSES.

(30) Priority: **12.02.79 US 11238**
**29.05.79 US 43548**
**23.01.80 US 114627**

(43) Date of publication of application: **18.02.81 Bulletin 81/07**

(45) Publication of the grant of the patent: **14.08.85 Bulletin 85/33**

(84) Designated Contracting States: **FR**

(73) Proprietor: **Exxon Research and Engineering Company**
**P.O.Box 390 180 Park Avenue**
**Florham Park New Jersey 07932 (US)**

(72) Inventor: **MITCHELL, Howard Lee III.**
**5188 Highland Road**
**Baton Rouge, LA 70808 (US)**

(74) Representative: **Pitkin, Robert Wilfred et al**
**ESSO Engineering (Europe) Ltd. Patents & Licences Apex Tower High Street**
**New Malden Surrey KT3 4DJ (GB)**

(56) References cited:
DE-B-1 160 855
GB-A- 859 391
GB-A-1 372 189
US-A-3 239 566
US-A-3 487 112
US-A-3 657 298
US-A-3 726 809
US-A-3 729 498
US-A-3 847 997
US-A-3 852 328
US-A-3 857 900

(56) References cited:
US-A-3 907 852
US-A-3 987 009
US-A-4 010 216
US-A-4 072 720
US-A-4 089 881
US-A-4 089 890
US-A-4 107 079
US-A-4 119 652
US-A-4 138 420
US-A-4 152 344

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 023 923 B1

US-A-4 157 313
US-A-4 169 861
US-A-4 179 401
US-A-4 193 943
US-A-4 201 714

SOVIET INVENTIONS ILLUSTRATED-Section Chemical-Derwent Publication Ltd., Week U44 (1973)
CHEMICAL ABSTRACTS, vol. 85, no. 14, October 4, 1976, Page 471, abstract 108734m Columbus, Ohio, USA, LEMMON, D.H. et al. "The syntheses and mass spectra of tris (trifluoropropynyl)phosphine,-arsine, and -stibine"
CHEMICAL ABSTRACTS, vol. 80, no. 19, May 13, 1974 page 356, abstract 107801r Columbus, Ohio, USA MINGALEVA, K.S. et al. "Dipole moments and structure of alkynylphosphines and derivatives of alkynylphosphorous acids"
JOURNAL OF ORGANOMETALLIC CHEMISTRY vol. 50, no. 1, March 16, 1973, H.A. PATEL et al. "Coordination complexes of acetylenic phosphines and diphosphines. V. Acetylene bridged derivatives of dicobalt octacarbonyl", pages 247-263
TETRAHEDRON, vol. 27, no. 16, August 1971 ROSENBERG, D. et al. "Interactions in acetylenes, an NMR approach", pages 3893-3907
CHEMICAL COMMUNICATIONS, no. 23, December 4, 1968 A.J. CARTY et al. "Evidence for metal-phosphorus dpi-dpi bonding from gamma(C=C) raman shifts in complexes of bis (diphenylphosphino) acetylene", pages 1559-61

**Description**

The invention relates to ligand materials, solvents, and complexes used in hydrocarbon and carbon monoxide conversion reactions. More specifically, the invention relates to novel solvents, ligand materials, complexes, ion-exchanged ligand compositions and photoreactive compositions and to processes of using these materials, for example, in hydrogenation, dehydrogenation, hydrocarbonylation, hydrocarbon synthesis, alcohol synthesis and water-gas disproportionation catalysis.

Various ligands and catalysts are known for hydrocarbon and carbon monoxide conversion reactions. For example, rhodium catalysts containing triaryl phosphine ligands have been extensively used for hydroformylation reactions. Likewise, cobalt hydroformylation catalysis has been widely used commercially, but the addition of phosphine ligands has proven of only minor value in such reactions.

Transition metal complexes of both triphenylphosphine and trialkyl phosphine have been widely studied as catalysts for hydroformylation, hydrogenation, etc. For their application in reactions of carbon monoxide, particularly carbonylations, see the monograph of Juergen Falbe, "Carbon Monoxide in Organic synthesis", Springer Verlag, New York, 1970. Also, general discussion of catalysis of reactions of carbon monoxide is included in "Homogeneous Catalysis Involving Carbon Monoxide" *Catalysis*, Vol. I, *Specialist Periodical Reports V,* The Chemical Society, Burlington House, London, 1977 by Davidson, et al. In the area of rhodium catalyzed hydroformylations of alpha-olefins, homogeneous catalyst systems employing triaryl phosphine and other trivalent phosphorus compounds in complex with rhodium plus excess phosphine ligand were described by R. L. Pruett and J. A. Smith in U.S. Patent No. 3,527,809.

Certain transition metal complexes containing phosphines covalently anchored to polymeric substrates have also been disclosed as heterogeneous catalyst systems. Such polymer-anchored complexes were reviewed by C. C. Leznoff in Vol. III, pp. 65—85, of the *Chemical Society Review* in 1974. The polymer-anchored rhodium hydroformylation catalysts were also discussed in detail in the *Journal of Organometallic Chemistry,* Vol. 134, pp. 85—94, in 1977 by W. H. Lang, A. T. Jurezicz, W. O. Haag, D. D. Whitehurst and L. D. Rollmann. Other complexes covalently anchored to inorganic solids, such as silica, were disclosed in a number of U.S. Patents such as No. 3,726,809 by K. G. Allum, S. McKenzie and R. C. Pitkethly and No. 4,151,114 by A. A. Oswald and L. L. Murrell.

Oswald in U.S. Patents Nos. 4,136,103 and 3,929,849 discloses tetraalkylphosphonium aluminosilicates and complexes thereof with group VIII transition metals, e.g. rhodium. There is no suggestion in these patents of using such catalyst in any hydrocarbon and carbon monoxide conversion processes.

Still other patents have described bis-phosphine compounds as complexes for rhodium. For example, Booth in U.S. Patents Nos. 3,965,192 and 3,560,539 discloses ethylene bis-(diphenylphosphine) as a ligand for rhodium complexes.

McVicker in U.S. Patent Nos. 3,939,188 and 3,946,082 discloses processes for preparing oxygenated products such as aldehydes and suggests as catalysts for his process zero valent rhodium complexes with various ligands thereon. As ligands for such complexes, McVicker suggests, for example, phosphine ligands with various substituents selected from a long list of possibilities among which are included halides such as fluoride, alkyl, alkoxy, cycloalkyl, cycloalkoxy, phenyl, phenyl substituted with halide, phenyl substituted with cycloalkyl, phenyl substituted with alkoxy, oxyphenyl, oxyphenyl substituted with alkyl, oxyphenyl substituted with cycloalkyl, oxyphenyl substituted with halide and oxyphenyl substituted with cycloalkoxy.

Kawse in U.S. Patent No. 4,013,700 discloses a process for the manufacture of polyhydric alcohols and their ether and ester derivatives by reacting oxides of carbon and hydrogen in the presence of small amounts of a quaternary phosphonium cation and a rhodium carbonyl complex. Exemplary quaternary phosphonium cations for the Kawse process are described in Column 4, lines 8—41 of the patent.

Some of the latest advancements in Fischer-Tropsch and water-gas shift reactions are disclosed in "Advances in Fischer-Tropsch Chemistry" by M. E. Dry in *Ind. Eng. Chem., Prod. Res. Dev.,* Vol. 15, No. 4, 1976; "Reductions with Carbon Monoxide and Water in Place of Hydrogen. 1. Hydroformylation Reaction and Water-Gas Shift" by H. C. Kang, et al in *Journal of the American Chemical Society*, Vol. 99, pp. 8323—8324 (1977); and "Coal Research Shifts to Soluble Catalysts" in *Chemical Week*, April 19, 1978, pp. 63 and 65. Advances in the use of silicate clusters are discussed in "New Silicate Has Cluster of Uses", *Chemical Week*, March 7, 1979, pp. 37 and 38. Various aspects of the support of metal complex catalysts on metal oxide supports as well as the use of such materials in photo reactions are discussed in "Chemical Modification of a Titanium(IV) Oxide ELectrode to Give Stable Dye Sensitisation Without a Supersensitiser" by S. Anderson, et al in *Nature*, Vol. 280, August 16, 1979 and "Photo-electrochemical Conversion of Optical Energy to Electricity and Fuels", by M. S. Wrighton in *Accounts of Chemical Research*, Vol. 12, No. 9, pp. 303—310 (September, 1979). Other aspects of Fischer-Tropsch synthesis and similar reactions are discussed in "Carbon Monoxide-Hydrogen Reactions", by H. Pichler, *Kirk Othmer's Encyclopedia of Chemical Technology* (Second Edition), Vol. 4, pp. 446—489 (1964).

In accordance with the present invention, a series of novel solvents, ligand materials and transition metal complexes have been discovered which are particularly advantageous for use in catalytic conversion processes for the conversion of hydrocarbons and carbon monoxide to more desirable products.

One aspect of the present invention involves compositions comprising compounds containing ethynyl linkages. These ethynyl compounds are of the formula

$$R_{4-b}L^{+}[QC{\equiv}C\text{+}J)]_{c}[\text{—}Q\text{+}J)_{a}]_{d} \qquad (I)$$

and

$$R_{3-b'}L[C{\equiv}CQ\text{+}J)_{a'}]_{c'}[\text{—}Q\text{+}J)_{a'}]_{d'} \qquad II$$

In these formulae, the symbols a and a' are 0, 1 or 2, while b is an integer of from 1 to 4 and b' is 1, 2 or 3. The symbol c is an integer of from 1 to 4, while c' is an integer of from 1 to 3. The symbol d is 0, 1, 2 or 3, and d' is 0, 1 or 2. The sum represented by c+d equals b and the sum represented by c'+d' equals b'. Each L is independently selected from a trivalent P or As atom. Each R group is independently selected from alkyl, aryl, alkoxyalkyl, aryloxyalkyl, alkoxyaryl, aryloxyaryl, ferrocenyl, and fluoro groups, wherein the aryl portions thereof can be substituted with a halogen and the alkyl portions thereof can be substituted with a member selected from halogen and hydroxy groups, provided that said halogens on said alkyl groups are not in an alpha-position with respect to L atoms unless they are fluoro atoms. Each Q group is independently selected from a covalent bond alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with a member selected from halogen and hydroxy groups and wherein the Q groups have a+1 points of attachment to L atoms. Each J is selected from $LR_2$ and $L^+R_3$. It is necessary for at least one R group on the L atom or atoms (but not $L^+$ atoms) to be a fluoro group. The compositions also include if necessary single or multiply charged inorganic or organic soluble anions having charges sufficient to balance the charges from any $L^+$ moieties.

In a preferred embodiment of this class of compounds, L in the formula II is a phosphorus atom. Particularly preferred ethynyl compounds are of the formulae

$$R_{3-b'}L\text{+}C{\equiv}C\text{—}QL^{+}R_{3})_{b'} \qquad (III)$$

and

$$R_{3-b'}P\text{+}C{\equiv}C\text{—}QP^{+}R_{3})_{b'} \qquad (IV)$$

wherein b' is an integer from 1 to 3 and R, L and Q are as defined above.

In another preferred embodiment of the invention, at least one R group on the L atom of formula II is a fluoro group. Particularly preferred ethynyl compounds within this class include compounds of the formulae

$$R_{2-b'}FL\text{+}C{\equiv}C\text{—}QP^{+}R_{3})_{b'} \qquad (V)$$

and

$$R_{2-b'}FP\text{+}C{\equiv}(\text{—}QP^{+}R_{3})_{b'} \qquad (VI)$$

wherein b' is an integer of from 1 to 2 and R, L and Q are as defined above.

Preferably, the compounds of formulae I and II contain from 1 to 3 $L^+$ moieties. In another preferred embodiment, the compounds of I and II contain at least one $L^+$ moiety attached through an ethynyl linkage to an L moiety. In yet another preferred embodiment of the invention involving ethynyl compounds, only one R group attached to an $L^+$ in the compounds of formulae I and II can be a methyl group, unless L of $L^+$ is nitrogen.

The anions balancing the charges from any $L^+$ moieties on the ethynyl compounds of the formulae I and II should preferably be non-nucleophilic anions such as carboxylates, phenolates, or halides (the term "halides" as used in this specification and in the attached claims is intended to include $F^-$, $Cl^-$ or $Br^-$ only), more preferably $CH_3SO_3^-$, $PhSO_3^-$, $PhPO_3^{=}$, $Ph_2PO_2^-$, $SO_4^{=}$, $PO_4^{-3}$ or $BF_4^-$, and most preferably $SbF_6^-$, $F_3CSO_3^-$, $TaF_6^-$, $(SbF_5O_3SCF_3)^-$, $(n\text{—}C_4H_9)_4B^-$, or $Ph_4B^-$. The more polar solvents, such as water, acetic acid, dimethylsulfoxide, $CH_3SO_3H$, $PhSO_3H$, $F_3CCO_2H$ or propionic acid, render the anions less nucleophilic, and therefore, even the more nucleophilic of the above anions becomes best suited for use in any desired application. This is especially true of $F^-$, $Cl^-$ and $Br^-$.

The compounds of formulae I and II should contain less than 200 atoms other than hydrogen atoms and halide atoms. Preferably these ethynyl compounds contain less than 100 atoms other than hydrogen and halide, more preferably, they contain less than 75 atoms other than hydrogen and halide, and most preferably, they contain less than 50 such atoms. This count of atoms in the ethynyl compounds does not include those atoms which are part of the anions associated with said compounds.

Suitable compounds of formula I which have been prepared include

(2) (diphenylmethylphosphonium) (difluorophosphino)ethyne methanesulfonate,

$$[Ph_2(CH_3)P^{\oplus}C{\equiv}CPF_2{}^{\ominus}O_3SCH_3]; \text{ and}$$

(3) ((diethylmethylphosphonium)ethynyl)difluorophosphine trifluoromethanesulfonate,

$$[(C_2H_5)_2(CH_3)P^{\oplus}C{\equiv}CPF_2{}^{\ominus}O_3SCF_3];$$

Other suitable compounds of the formula I containing ethynyl linkages between the heteroatom L and the quaternized heteroatom $L^+$ include

(6) (2-ferrocenyl)ethynyl)((para-methoxy-phenyl)ethynyl)(2-triethylphosphonium)ethynyl)arsine tetrabutylboranate,

$$[(((C_5H_5)Fe(II)(C_5H_4))C{\equiv}C\text{—})(p\text{—}CH_3O(C_6H_4\text{—}C{\equiv}C\text{—})((C_2H_5)_3P^{\oplus}C{\equiv}C\text{—})As(n\text{—}C_4H_9)_4B^{\ominus}]; \text{ and}$$

(7) (2-(trimethylammonium)ethynyl)(ferrocenyl)(2-(triphenylarsonium)ethynyl)phosphine 1,4- bis(tributylboranate)butane,

$$[((CH_3)_3N^{\oplus}C{\equiv}C\text{—})((C_5H_5)Fe(C_5H_4))(Ph_3{}^{\oplus}AsC{\equiv}C\text{—})P((n\text{—}C_4H_9)_3B^{\ominus}CH_2CH_2\text{—})_2].$$

Suitable non-salt ethynyl compounds included with the scope of formula II which have been prepared include

(13) (diphenylphosphino) (difluorophosphino)ethyne,

$$[Ph_2PC{\equiv}CPF_2]; \text{ and}$$

(15) (diethylphosphino)(difluorophosphino)ethyne,

$$[(C_2H_5)_2PC{\equiv}CPF_2].$$

Other suitable compounds of formula II are

(16) bis(2-(pentafluorophenyl)ethylnyl)fluorophosphine,

$$[((C_6F_5)C{\equiv}C\text{—})_2PF];$$

(17) bis(2-(trifluoromethyl)ethynyl)fluoroarsine,

$$[(CF_3C{\equiv}C\text{—})_2AsF];$$

(18) (2-(bis(tertiary-butyl)amino)ethynyl)(2-phenoxyethyl)fluoroarsine,

$$[((((CH_3)_3C)_2N)C{\equiv}C\text{—})(C_6H_5OCH_2CH_2\text{—})AsF];$$

(19) (2-(pentafluorophenyl)ethynyl)(trifluoromethyl)fluorophosphine,

$$[(CF_3)((C_6F_5)C{\equiv}C\text{—})PF];$$

(20) bis(ferrocenyl)(2-(pentafluorophenyl)ethynyl)arsine,

$$[((C_5H_5)Fe(C_5H_4))_2((C_6F_5)C{\equiv}C\text{—})As];$$

(21) tris(2-(trifluoromethyl)ethynyl)phosphine,

$$[(CF_3C{\equiv}C\text{—})_3P]; \text{ and}$$

(22) tris(2-(pentafluorophenyl)ethynyl)phosphine,

$$[(C_6F_5C{\equiv}C\text{—})_3P].$$

Another class of preferred ethynyl compounds are compounds of the formula

$$R^1_{2-b}F_bL\text{—}C{\equiv}CR^1 \qquad \text{(VII)}$$

In this formula III, b represents an integer of 1 or 2. Each $R^1$ group is independently selected from the group consisting of an R group and a group $Q(J)_a$, wherein each R group is independently selected from alkyl, aryl, alkoxyalkyl, aryloxyalkyl, alkoxyaryl, aryloxyaryl, ferrocenyl, and fluoro groups, wherein the aryl portions thereof can be substituted with a halogen and the alkyl portions thereof can be substituted with a member selected from halogen and hydroxy groups, provided that the halogens on the alkyl groups are not in an alpha-position with respect to the L atoms unless they are fluoro atoms. Each Q group is independently selected from alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with a member selected from halogen and hydroxy groups, wherein said Q groups have a+1 points of attachment to L atoms. The symbol J is selected from $LR_2$ and $L^+R_3$ wherein R is

defined as hereinabove. These compounds include single or multiply charged inorganic or organic, soluble anions having charges sufficient to balance the charges from any $L^+$ moieties on the compounds. Exemplary of suitable compounds within this class are

(23) Bis(2-(pentafluorophenyl)ethynyl)fluorophosphine,

$$[((C_6F_5)C{\equiv}C—)_2PF];$$

(24) Bis(2-(trifluoromethyl)ethynyl)fluoroarsine,

$$[(CF_3C{\equiv}C—)_2AsF];$$

(25) (2-(bis(tertiarybutyl)amino)ethynyl)(2-phenoxyethyl)fluoroarsine,

$$[(((CH_3)_3C)_2N)C{\equiv}C—)(C_6H_5OCH_2CH_2—)AsF];$$ and

(26) (2-(pentafluorophenyl)ethynyl)(trifluoromethyl)fluorophosphine,

$$[(CF_3)((C_6F_5)C{\equiv}C—)PF].$$

Another aspect of the present invention relates to a complex VIII comprising a Group VIB, VIIB, VIII or IB transition metal in complex association with at least one ligand selected from ethynyl compounds of the formulae I, II, and III, IV, V, VI and VII, wherein the ethynyl compounds contain at least one non-quaternized L atom.

Transition metals for general use in the complexes of formula VIII and the other transition metal complexes described in further detail below include mono-atomic and clusters comprising transition metal atoms. The particular transition metal atom or cluster chosen for use in any particular process depends upon a number of factors, including the conditions of the process being performed and the properties needed for the complexes in the desired process, e.g., certain transition metals are better for use as hydroformylation catalysts than as, say, hydrogenation catalysts. The terminology "transition metal cluster", as used in this specification and the attached claims, is meant to include metal clusters comprising at least one transition metal atom covalently bonded to a member selected from the group consisting of (1) at least one transition metal atom, (2) a group covalently bonded to at least one transition metal atom, which group renders the transition metal atoms thereby joined electronically contiguous, and (3) combinations of (1) and (2) above. Examples of such transition metal clusters (enumerated without specifying the associated ligands thereto) include, e.g.. $Ru_3$, $Rh_6$, $Fe_3$, $Co_6$, $Mo_2$, $Re_2$, $Pt_3$, $Co_6$, and $Rh_4$. Suitable atoms for covalently bonding at least two transition metal atoms in the clusters, which atoms render the transition metal atoms thereby joined electronically contiguous, include Sn, N, P, H, C, and occasionally O or S.

Among the preferred ligands for use in the complexes of the formula VIII are included the preferred ethynyl compounds described in greater detail above. Thus, exemplary of suitable complexes of formula VIII which were synthesized, include

(27) (tris((diphenylmethylphosphonium)ethynyl)phosphine)tetracarbonyliron(O) tris(methanesulfonate),

$$[((Ph_2(CH_3)P^{\oplus}C{\equiv}C)_3P)Fe(CO_4(CH_3SO_3{}^{\ominus})_3];$$

(28) ((diphenylmethylphosphonium)(diphenylphosphino)ethyne)tetracarbonyliron(O) methanesulfonate,

$$[(Ph_2(CH_3)P^{\oplus}C{\equiv}CPPh_2)—Fe(CO)_4CH_3SO_3{}^{\ominus}];$$

(29) (tris((diethylmethylphosphonium)ethynyl)-phosphine)tetracarbonyliron(O) tris(methanesulfonate),

$$[((C_2H_5)_2(CH_3)P^{\oplus}C{\equiv}C—P)_3(Fe(CO)_4({}^{\ominus}O_3SCH_3)_3];$$

(30) bis(tris((diethylmethylphosphonium)ethynyl)phosphine)(acetylacetonato)-rhodium(I) hexakis(methanesulfonate),

$$[((C_2H_5)_2(CH_3)P^{\oplus}C{\equiv}C)—_3P)_2Rh—(C_5H_7O_2)(CH_3SO_3{}^{\ominus})_6];$$

(31) tetrakis(tris((diethylmethylphosphonium)ethynyl)phosphine)platinum(O) dodecakis(methanesulfonate),

$$[((C_2H_5)_2(CH_3)P^{\oplus}C{\equiv}C—)_3P)_4Pt^{o}(CH_3SO_3{}^{\ominus})_{12}];$$

(32) bis(tris((diethylmethylphosphonium)ethynyl)phosphine)dichloroplatinum(II) hexakis(methanesulfonate),

$$[((C_2H_5)_2(CH_3)P^{\oplus}—C{\equiv}C—)_3P_2PtCl_2(CH_3SO_3^{\ominus})_6];$$

(33) tris((diphenylmethylphosphonium)ethynyl)difluorophosphine)carbonylhydridorhodium(I) tris(methanesulfonate),

$$[(Ph_2(CH_3)P^{\oplus}C{\equiv}CPF_2)_3Rh(CO)H(^{\ominus}O_3SCH_3)_3];$$

(34) tris(((diphenylmethylphosphonium)ethynyl)(difluorophosphine)carbonylhydridoiridium(I) tris(methanesulfonate),

$$[(Ph_2(CH_3)P^{\oplus}C{\equiv}CPF_2)_3Ir(CO)H(^{\ominus}O_3SCH_3)_3];$$

(35) tris(((diethylmethylphosphonium)ethynyl)difluorophosphine)carbonylhydridorhodium(I) tris(trifluoromethanesulfonate),

$$[(C_2H_5)_2(CH_3)P^{\oplus}C{\equiv}C—PF_2)_3Rh(CO)H(^{\ominus}O_3SCF_3)_3];$$

(36) (((diphenylmethylphosphonium)ethynyl)difluorophosphine)tetracarbonyliron(O) methanesulfonate,

$$[(Ph_2(CH_3)P^{\oplus}C{\equiv}CPF_2)Fe(CO)_4{}^{\ominus}O_3SCH_3];$$

(37) (((diethylmethylphosphonium)ethynyl)difluorophosphine)tetracarbonyliron(O) trifluoromethanesulfonate,

$$[(C_2H_5)_2(CH_3)P^{\oplus}C{\equiv}CPF_2)Fe(CO)_4{}^{\ominus}O_3SCF_3];$$

(38) tris(tris((diphenylmethylphosphonium)ethynyl)phosphine)carbonylhydridorhodium(I) nonakis(methanesulfonate),

$[((Ph_2(CH_3)P^{\oplus}C{\equiv}C)—_3P)_3Rh(CO)H(CH_3SO_3^{\ominus})_9]$; and

(39) tris((diphenylmethylphosphonium)ethynyl)phosphine)tetracarbonyliron(O) tris(methanesulfonate),

$$[((C_2H_5)_2(CH_3)P^{\oplus}C{\equiv}C—)_3P)Fe(CO)_4(^{\ominus}O_3SCH_3)_3].$$

Other suitable complexes of formula VIII are

(40) bis(tris(2-diphenylphosphino)ethynyl)phenylphosphonium)decacarbonylhexacobalt(O) 1,8-octanedisulfonate,

$$[((Ph_2PC{\equiv}C—)_3P^{\oplus}Ph)_2Co_6(CO)_{10}(—CH_2CH_2CH_2CH_2SO_3^{\ominus})_2];$$

(41) bis((2-(bis(2-methoxyethyl)phosphino)ethynyl)bis(2-methoxyethyl)methylphosphonium))tetracarbonylchromium(O) oxalate,

$$[((CH_3OCH_2CH_2)_2PC{\equiv}CP^{\oplus}(CH_2CH_2OCH_3)_2(CH_3))_2Cr(CO)_4C_2O_4{}^{=}];$$

(42) ((2-(pentafluorophenyl)ethynyl)difluoroarsine)hexakis(tertiary-butylisocyano)tetranickel(O),

$$[(C_6F_5C{\equiv}C—AsF_2)Ni_4(CNC(CH_3)_3)_6];$$

(43) ((tertiarybutyl)(2-para-methoxyphenyl)ethynyl)fluorophosphine)nonacarbonyldirhenium(O),

$$[(((CH_3)_3C)(p—CH_3O—(C_6H_4)C{\equiv}C—)PF)Re_2(CO)_9];$$

(44) (cyclopentadienyl)(dicarbonyl)(2-(bis(4-hydroxybutyl)methylphosphonium)ethynyl)difluorophosphine)manganese(I) tetrakis(pentafluoropheny)boranate,

$$[(C_5H_5)Mn(CO)_2(PF_2(—C{\equiv}C—P^{\oplus}(CH_3)(CH_2CH_2CH_2CH_2OH)_2))(C_6F_5)_4B^{\ominus}];$$

(45) (cyclopentadienyl)(carbonyl)(((trifluoromethyl)ethynyl)difluorophosphine)bromoiron(II),

$[((C_5H_5)FeBr(CO)(PF_2(—C{\equiv}C—CF_3))]$; and

(46) bis(((ferrocenylethynyl)(2-triethylphosphonium)ethynyl)fluorophosphine)(tri-n- butylphosphine) (triphenylarsine)(triethylphosphite)octacarbonyltriruthenium(O) benzenephosphonate,

$$[(((((C_5H_5)Fe(II)(C_5H_4))C{\equiv}C—)((C_2H_5)_3P^{\oplus}C{\equiv}C—)PF)((n—C_4H_9)_3P)(Ph_3As)((C_2H_5O)_3P)Ru_3(CO)_8)_2PhPO_3^{=}]$$

These compounds and complexes of formula I—VIII are useful as solvents and co-solvents and as catalysts in catalytic conversion processes for the conversion of hydrocarbons and carbon monoxide, to desired products, e.g., hydrogenation using as reagents either hydrogen or carbon monoxide and water, dehydrogenation, hydrocarbonylation of olefin, hydrocarbon synthesis, alcohol synthesis, and water-gas disproportionation catalysts (sometimes called water-gas shift. The choice of the particular compound and/or complex depends upon the process desired and the conditions of such process under which it is to be run.

Still another preferred embodiment of the present invention relates to salt ligand complexes of formula XVI comprising a group VIB, VIIB, VIII or IB transition metal in complex association with a ligand selected from compounds with structures with the formula

$$R_{4-b}L^+[—Q{+}J)_a]_b \qquad XVII$$

and

$$R_{3-b'}L[—Q{+}J)_{a'}]_{b'} \qquad XVIII$$

In the complex of formula XVII and XVIII, b is an integer of from 1 to 4, while b' is an integer of from 1 to 3. the symbols a and a' each represent 0, 1 or 2. Each L is independently selected from P and As, J is $LR_2$ or $L^+R_3$. Each R group is independently selected from alkyl, aryl, alkoxyalkyl, aryloxyalkyl, alkoxyaryl, aryloxyaryl, ferrocenyl and fluoro groups, wherein the aryl portions thereof can be substituted with a halogen and the alkyl portions thereof can be substituted with a halogen and/or hydroxy group, provided that said halogens on said alkyl groups are not in an alpha-position with respect to said L atom unless they are fluoro atoms. When a is zero, each Q group is independently selected from alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with a halogen and/or a hydroxy group and wherein said Q groups have a+1 points of attachment to L atoms. When a is not zero, Q is a corresponding divalent radical having a+1 points of attachment to L atoms. The compounds of formulae XVII and XVIII also include single or multiply charged inorganic or organic, soluble anions having charges sufficient to balance the charges from any $L^+$ moieties on the compounds. These ligands of formulae XVII and XVIII contain at least one other L atom which is a quaternised $L^+$ and at least one other L atom which is a non-quaternised L. The complex also has to contain a carbonyl group or an acetylaceto group. When Q is alkylene, at least one R attached to $L^+$ must be aryl.

In the compounds of formulae XVII and XVIII, L preferably represents phosphorus, both in the L and $L^+$ moieties. The number of $L^+$ moieties in the compounds of I and II is preferably a maximum of 3, more preferably 2 and most preferably 1. The total number of non-hydrogen and non-halide atoms in the compounds of formulas I and II is preferably less than 200, more preferably less than 150 and most preferably less than 75.

Suitable ligands of formulae XVII and XVIII which have been prepared include

(54) (2-(diphenylphosphino)ethyl)diphenylmethylphosphonium methanesulfonate,

$$[Ph_2P^{\oplus}(CH_3)CH_2CH_2PPh_2{}^{\ominus}O_3SCH_3];$$

(55) tris((diphenylmethylphosphonium)ethynyl)phosphine tris(methanesulfonate),

$$[(Ph_2(CH_3)P^{\oplus}C{\equiv}C{+}_3P(^{\ominus}O_3SCH_3)_3];$$

(56) (2-diphenylphosphino)ethynyl)diphenylmethylphosphonium methanesulfonate,

$$[Ph_2(CH_3)P^{\oplus}C{\equiv}CPPh_2{}^{\ominus}O_3SCH_3];$$

(57) (2-(diphenylphosphino)-trans-ethenyl)diphenylmethylphosphonium methanesulfonate,

$$[Ph_2P—t—CH{=}CH—^{\oplus}P(CH_3)Ph_2{}^{\ominus}O_3SCH_3];$$

(58) tris((diethylmethylphosphonium)ethynyl)phosphine tris(methanesulfonate),

$$[((C_2H_5)_2(CH_3)P^{\oplus}C{\equiv}C{+}_3P(^{\ominus}O_3SCH_3)_3];$$

(59) ((difluorophosphino)ethynyl)diphenylmethylphosphonium methanesulfonate,

$$[Ph_2(CH_3)P^{\oplus}C{\equiv}CPF_2{}^{\ominus}O_3SCH_3];$$

(60) ((difluorophosphino)ethynyl)diethylmethylphosphonium trifluoromethanesulfonate,

$[(C_2H_5)_2(CH_3)P^{\oplus}C{\equiv}C{-}PF_2CF_3SO_3^{\ominus}]$;

(62) (2-(diphenylarsino)ethyl)diphenylmethylphosphonium methanesulfonate,

$[Ph_2AsCH_2CH_2P^{\oplus}(CH_3)Ph_2{}^{\ominus}O_3SCH_3]$;

(63) 1-(diphenylphosphino)-1'-(diphenylmethylphosphonium)ferrocene(II) methanesulfonate,

$[Ph_2P(C_5H_4)Fe(II)(C_5H_4P^{\oplus}(CH_3)Ph_2{}^{\ominus}O_3SCH_3]$;

(64) tris(para-trimethylammoniumphenyl)phosphine tris(benzenesulfonate),

$[((CH_3)_3N^{\oplus}(p{-}C_6H_4))_3P(PhSO_3^{\ominus})_3]$;

(65) bis(para-trimethylammoniumphenyl)(para-dimethylaminophenyl)phosphine bis(benzenesulfonate),

$[((CH_3)_3N^{\oplus}(p{-}C_6H_4))_2((CH_3)_2N(p{-}C_6H_4))P(PhSO_3^{\ominus})_2$;

(66) bis(para-dimethylaminophenyl)(paratrimethylammoniumphenyl)phosphine benzenesulfonate,

$[[((CH_3)_2N(p{-}C_6H_4))_2((CH_3)_3N^{\oplus}(p{-}C_6H_4))P(PhSO_3^{\ominus})]$;

(69) bis(2-(diphenylphosphino)ethyl)methylphosphonium methanesulfonate,

$[(Ph_2PCH_2CH_2)_2P^{\oplus}(CH_3)PhCH_3SO_3^{\ominus}]$;

(73) (2-(dipyenylphosphino)ethyl)diphenylmethylphosphonium trifluoromethanesulfonate,

$[Ph_2PCH_2CH_2P^{\oplus}(CH_3)Ph_2CF_3SO_3^{\ominus}]$;

(74) (4-(diphenylphosphino)butyl)diphenylmethylphosphonium methanesulfonate,

$[Ph_2PCH_2CH_2CH_2CH_2P^{\oplus}(CH_3)Ph_2CH_3SO_3^{\ominus}$;

(75) (6-(diphenylphosphino)hexyl)diphenylmethylphosphonium methanesulfonate,

$[Ph_2P(CH_2)_6P^{\oplus}CH_3Ph_2CH_3SO_3^{\ominus}]$;

(77) 2,2-bis((diphenylphosphine)methylene)(methyl)(diphenylmethylphosphonium)methylene)methane methanesulfonate,

$[(Ph_2PCH_2)_2(CH_3)CCH_2P^{\oplus}(CH_3)Ph_2CH_3SO_3^{\ominus}]$; and

(78) (2-(diphenylphosphino)ethyl)(diphenyl)(3-methylbutyl)phosphonium formate,

$[Ph_2PCH_2CH_2P^{\oplus}(CH_2CH_2CH(CH_3)_2)Ph_2HCO_2^{\ominus}]$.

These listed ligand materials are typical of the types of ligands used in the formation of the new complex compositions below. Other similar ligands can also be employed in such complexes.

These ligands of formulae XVII and XVIII are useful as solvents and cosolvents for various catalytic conversion processes of hydrocarbons and carbon monoxide. For example, they can be employed as solvents in a catalytic conversion process comprising contacting a reactant selected from hydrocarbon, hydrocarbon precursors, and mixtures of carbon oxides and water with a catalyst so as to catalytically convert the reactant to the desired product. In such a process the complex of formula XVII and/or XVIII may be used with a cosolvent with which it is miscible, wherein the cosolvent preferably has a pH of greater than about 6.5. Preferably, the complex of formula XVII or XVIII is the same as the ligand complex with the catalyst. Examples of such catalytic conversion processes include hydrogenation, dehydrogenation, hydrocarbon synthesis, hydrocarbonylation of olefins or water-gas disproportionation catalysts. In these processes, a catalyst suitable to provide the desired reaction is, of course, employed.

The transition metals of the complexes of formula XVI include single transition metal atoms and clusters thereof as discussed above. The choice of the transition metal, like the choice of ligand, is dependent upon a number of factors including the properties of the ligand attached to the transition metal,

the process in which the complex will be used and the conditions of such process, and the costs of the various ligands, metals, catalysts, solvents, etc.

Complexes of formula XVI which were prepared include

(79) Tris(2-(diphenylphosphino)ethyl)diphenylmethylphosonium)carbonylhydridorhodium(I) tris(methanesulfonate,

$$[(PhP^{\oplus}(CH_3CH_2CH_2PPh_2)_3Rh(CO)H(^{\ominus}O_3SCH_3)_3];$$

(80) (Tris(2-(diphenylmethylphosphonium)ethynyl)phosphine)tetracarbonyliron(O) tris(methanesulfonate),

$$[(Ph_2(CH_3)P^{\oplus}C{\equiv}C{-})_3P)Fe(CO)_4(^{\ominus}O_3SCH_3)_3)];$$

(81) (2-diphenylphosphinoethynyl)diphenylmethylphosphonium)tetracarbonyliron(O) methanesulfonate,

$$[(Ph_2(CH_3)P^{\oplus}C{\equiv}CPPh_2)Fe(CO)_4{}^{\ominus}O_3SCH_3)];$$

(82) (Tris(2-(diethylmethylphosphonium)ethynyl)phosphine)tetracarbonyliron(O) tris(methanesulfonate),

$$[((C_2H_5)_2(CH_3)P^{\oplus}C{\equiv}C{-})_3P)Fe(CO)_4(^{\ominus}O_3SCH_3)_3)];$$

(83) Bis(tris(2-(diethylmethylphosphonium)ethynyl-phosphine)(acetylacetato)rhodium(I) hexakis(methanesulfonate),

$$[((C_2H_5)_2(CH_3)P^{\oplus}C{\equiv}C{-})_3P))_2Rh(C_5H_7O_2)(CH_3SO_3{}^{\ominus})_6];$$

(85) Bis(tris(2-(diethylmethylphosphonium)ethynyl)phosphine)dichloroplatinum(II) hexakis(methanesulfonate),

$$[((C_2H_5)_2(CH_3)P^{\oplus}C{\equiv}C{-})_3P)_2PtCl_2(^{\ominus}O_3SCH_3)_6];$$

(86) Tris((2-(difluorophosphino)ethynyl)diphenylmethylphosphonium)carbonylhydridorhodium(I) tris(methanesulfonate),

$$[(Ph_2(CH_3)P^{\oplus}C{\equiv}CPF_2)_3Rh(CO)H(^{\ominus}O_3SCH_3)_3];$$

(87) Tris(2-(difluorophosphino)ethynyl)diethylmethylphosphonium)carbonylhydridorhodium(I) tris(trifluoromethanesulfonate),

$$[(C_2H_5)_2(CH_3)P^{\oplus}C{\equiv}C{-}PF_2)_3Rh(CO)H(CF_3SO_3{}^{\ominus})_3];$$

(88) Tris(2-(difluorophosphino)ethynyl)diphenylmethylphosphonium)carbonylhydridoiridium(I) tris(methanesulfonate),

$$[(Ph_2(CH_3)P^{\oplus}C{\equiv}CPF_2)_3Ir(CO)H(^{\ominus}O_3SCH_3)_3)];$$

(90) Tris(2-(diphenylarsino)ethyl)diphenylmethylphosphonium)carbonylhydridorhodium(I) tris(methanesulfonate),

$$[(Ph_2AsCH_2CH_2P^{\oplus}(CH_3)Ph_2)_3Rh(CO)H(^{\ominus}O_3SCH_3)_3];$$

(91) Tris(1-(diphenylphosphino)-1′-diphenylmethylphosphonium)ferrocene(II)carbonylhydridorhodium(I) tris(methanesulfonate),

$$[(Ph_2P(C_5H_4)Fe(II)(C_5H_4)P^{\oplus}(CH_3)Ph_2)_3Rh(CO)H)^{\ominus}O_3SCH_3)_3];$$

(92) Tris(tris(para-(trimethylammonium)phenyl)phosphine)carbonylhydridorhodium(I) nonakis(benzenesulfonate),

$$[(((CH_3)_3N^{\oplus}(p{-}C_6H_4))_3P)_3Rh(CO)H(PhSO_3{}^{\ominus})_9];$$

(100) Tris(2-(diphenylphosphino)ethyl)diphenylmethylphosphonium)carbonylhydridoidridium(I) tris(methanesulfonate),

$$[(Ph_2P^{\oplus}(CH_3)CH_2CH_2PPh_2)_3Ir(CO)H(^{\ominus}O_3SCH_3)_3];$$

(103) Tetrakis((2-(diphenylphosphino)ethyl)diphenylmethylphosphonium)octacarbonyltetrairidium(O) tetrakis(methanesulfonate),

$$[(Ph_2P^{\oplus}(CH_3)CH_2CH_2PPh_2)_4Ir_4(CO)_8(^{\ominus}O_3SCH_3)_4];$$

(104) ((2-(diphenylphosphino)ethyl)diphenylmethylphosphonium)tetracarbonyliron(O) methanesulfonate,

$$[(Ph_2P^{\oplus}(CH_3)CH_2CH_2PPh_2)Fe(CO)_4{}^{\ominus}O_3SCH_3];$$

(105) Bis((2-(diphenylphosphino)ethyl)diphenylmethylphosphonium)tricarbonyliron(O) bis(methanesulfonate),

$$[(Ph_2P^{\oplus}(CH_3)CH_2CH_2PPh_2)_2Fe(CO)_3(^{\ominus}O_3SCH_3)_2];$$

(106) Hexakis((2-(diphenylphosphino)ethyl)diphenylmethylphosphonium)hexacarbonyltriruthenium(O) hexakis(methanesulfonate),

$$[(Ph_2P^{\oplus}(CH_3)CH_2CH_2PPh_2)_6Ru_3(CO)_6(CH_3SO_3{}^{\ominus})_6];$$

(107) Bis((2-(diphenylphosphino)ethyl)diphenylmethylphosphonium)bis(acetylacetate)ruthenium(II) bis(methanesulfonate),

$$[(Ph_2P^{\oplus}(CH_3)CH_2CH_2PPh_2)_2Ru(C_5H_7O_2)_2(CH_3SO_3{}^{\ominus})_2];$$

(108) Bis((2-(diphenylphosphino)ethyl)diphenylmethylphosphonium)carbonyldichlororuthenium(II) bis(methanesulfonate),

$$[(Ph_2P^{\oplus}(CH_3)CH_2CH_2PPh_2)_2Ru(CO)Cl_2(^{\ominus}O_3SCH_3)_2];$$

(109) Bis(triphenylphosphine)(((2-(diphenylphosphino)ethyl)diphenyl(3-methylbutyl)-phosphonium)carbonylhydridorhodium(I) formate,

$$[(Ph_3P)_2(Ph_2PCH_2CH_2{}^{\oplus}P(CH_2CH_2CH(CH_3)_2)Ph_2)Rh(CO)HCO_2{}^{\ominus}];$$

(110) Tris((2-(diphenylphosphino)ethyl)(diphenyl)(3- methylbutyl)phosphonium) carbonylhydridorhodium(I) tris(formate),

$$[(Ph_2PCH_2CH_2{}^{\oplus}P(CH_2CH_2CH(CH_3)_2)Ph_2)_3Rh(CO)H(HCO_2{}^{\ominus})_3];$$ and

(111) Tris((2-(diphenylphosphino)ethyl)(diphenyl)(3- methylbutyl)phosphonium) carbonylhydridorhodium(I) tribromide,

$$[(Ph_2PCH_2CH_2{}^{\oplus}P(CH_2CH_2CH(CH_3)_2)Ph_2)_3Rh(CO)H(Br^-)_3].$$

The structures and compositions of the listed complexes represent the wide variety of structures and compositions which are suitable for inclusion in these complexes, but other similar materials can also be employed. The complexes of formula XVI are useful as solvents and cosolvents, and as catalysts in catalytic conversion reactions of hydrocarbons and carbon monoxide, such as hydrogenation using, as reagents, $H_2$ or CO plus $H_2O$, dehydrogenation, hydrocarbonylation of olefins, hydrocarbon synthesis, alcohol synthesis and/or water-gas shift disproportionation catalysis.

Yet another aspect of the present invention relates to novel compositions of matter (XIX) comprising surface tetravalent metal oxide selected from $SiO_2$, $TiO_2$, $ZrO_2$, $HfO_2$, $ThO_2$, $GeO_2$, $SnO_2$ and mixtures thereof and with other metal oxides, and attached to the surface of said tetravalent metal oxide, a coordinating group of a formula selected from $-L^1R_2$(XX) and $-L^1R_{2-b}[Q(J)_a]_b$ wherein the coordinating group is attached to the tetravalent metal oxide through an oxide linkage. In these new compositions of matter (XIX), each $L^1$ atom is trivalent P or As. The symbol a is 1 or 2, while b is 1 or 2. Each R is independently selected from alkyl, aryl, alkoxyalkyl, aryloxyalkyl, alkoxy aryl, aryloxyaryl, ferrocenyl, and fluoro groups, wherein the aryl portions thereof can be substituted with a halogen and the alkyl portions thereof can be substituted with halogen groups, provided that said halogens on said alkyl groups are not in an alpha-position with respect to said L atom unless they are fluoro atoms. The symbol Q is a member selected from alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with halogen groups and wherein said Q group has a+1 points of attachment to L atoms. The group J is selected from $LR_2$ and $L^+R_3$, wherein L is independently selected from P and As. In these new compositions, L is preferably phosphorus, and the surface tetravalent metal oxide is preferably $SiO_2$ or $TiO_2$, depending

on the use to which the novel composition of formula XIX will be put and the conditions to which they will be subjected. The surface tetravalent metal oxide can exist as a coating on a substrate. The surface area of the surface tetravalent metal oxide is preferably greater than 5 square meters per gram, more preferably greater than 50 square meters per gram and most preferably greater than 100 square meters per gram. The average area occupied by each equivalent of L atom should preferably be less than 125 square angstroms per L atom, more preferably less than 100 and most preferably less than 75 square angstroms per equivalent of L atoms wherein the lower limit is the area occupied by a single L atom with a monolayer coverage. These last two statements are true of all the surface tetravalent metal oxide compositions included within the scope of the present invention discussed further below. The R groups on these tetravalent metal oxide compositions and the other tetravalent metal oxide compositions discussed further below should be thermally stable, i.e., they should not include any substituents which would render them thermally unstable such as hydroxy groups because of the methods of preparation of these compounds which involve heating to 200°C. or higher.

Examples of exemplary compounds within this class which were prepared include:

(112) titania-oxydiphenylphosphine,

$$[TiO_2/Ti—O—Ph/_2];$$

(113) n-titania-oxydiphenylphosphine,

$$[n—TiO_2/Ti—O—Ph/_2];$$ and

(114) silica-oxydiphenylphosphine,

$$[SiO_2/Si—O—Ph/_2]$$

Other suitable compounds within this class are

(115) Titania-oxy-bis(2-cyonoethyl)phosphine,

$$[TiO_2/Ti—O—P(CH_2CH_2CN)_2];$$

(116) Silica-oxy-bis(2-cyanoethyl)phosphine,

$$[SiO_2/Si—O—P(CH_2CH_2CN)_2];$$

(117) Titania-oxydiethylphosphine,

$$[TiO_2/Ti—O—P(CH_2CH_3)_2];$$

(118) Thoria-oxy-bis(2-phenylethynyl)phosphine,

$$[ThO_2/Th—O—P(C{\equiv}C—Ph)_2];$$

(119) Silica-oxy-bis(ferrocenyl)arsine,

$$[SiO_2/Si—O—As((C_5H_4)Fe(II)(C_5H_5))_2];$$

(120) Zirconia-oxybenzylphenylphosphine,

$$[ZrO_2/Zr—O—P(Ph)(CH_2Ph)];$$

(121) Titania-oxydifluorophosphine,

$$[TiO_2/Ti—O—PF_2];$$

(122) n-titania-oxy-(2-bis(diethylmethylphosphonium)ethynyl)phosphine benzenephosphonate,

$$[n—TiO_2/Ti—O—P(C{\equiv}CP^{\oplus}(CH_3)(C_2H_5)_2)_2(PhPO_3^{=})];$$

(123) n-titania-oxydifluorophosphine,

$$[n—TiO_2/Ti—OPF_2];$$

(124) silica-oxytrifluoromethylfluorophosphine,

$$[SiO_2/Si—O—PF(CF_3)];$$

(125) silica-titania mixed oxide-oxypentafluorophenylfluoroarsine,

$$[SiO_2—TiO_2/\text{"M"}—O—AsF(C_6F_5)];\ \text{and}$$

(126) alumina-silica mixed oxide-oxy(2-(tri-n-butyl)silyl)ethyl)fluorophosphine,

$$[Al_2O_3—\text{"M"}O_2/Si—O—PF(CH_2CH_2Si(n—C_4H_9)_3];$$

and other similar materials.

The composition of XIX can be used to trap metal, especially transition metals, from liquid or gas streams. They are also useful as intermediate or catalyst precursors in the production of metal complexes as shown below.

Still another aspect of the present invention involves a complex (XX) of a group VIB, VIIB, VIIIB, or IB transition metal in complex association with a ligand of formula XIX as defined above. As with the compounds of formula XIX described above, in the present complexes, the metal oxide comprises a semi-conductor or semi-conductor coated electrode. Preferably, the semi-conductor or semi-conductor coated electrode comprises an n-type $TiO_2$.

These solid ligand materials have as supports materials of low or high surface area in which the surface is a tetravalent metal oxide. The bulk material may or may not be the same material, it can be a metal or a metal oxide or alloy or mixtures of metal oxides which are coated with either the mixture or pure metal oxides such as, titanium dioxide, silicon dioxide, thoreum dioxide, zirconium dioxide, germanium dioxide or hafnium dioxide. It is possible to use tin dioxide as well but this is less preferable. In addition, the support material can be either a conductor, insulator, or semiconductor, and for some purposes, it can be partially reduced materials such as n-type titanium in which some of the titanium is reduced to titanium $Ti^{+3}$ to make the titanium dioxide into a semiconductor. The three examples actually employed were silicon dioxide or silica ($SiO_2$), titanium dioxide or titania ($TiO_2$), and (n—$TiO_2$) or semiconductor titania in which some of the titanium has been reduced. In the discussions and formulas that follow only the metal part of the support such as titanium Ti or silicon Si will be shown as the formula in showing the reactions with the structures with the materials on the surfaces.

The primary linkage which characterizes the material is a structure with an M-O-L linkage. The L atoms are specifically either phosphorus or arsenic in their trivalent states and in which the other two substituents on the phosphorus or arsenic are organic substituents, that is, they are bonded through a carbon to the atom L. The primary group thus attached on the support for the formation of such a ligand can be diphenylphosphine-oxy group. Thus, preferred ligand structures are $TiO_2/Ti/Ti—O—PR_2$, $SiO_2/Si—O—PR_2$ and n—$TiO_2/Ti—O—PR_2$.

These materials act as if there were phosphine ligands even though they have an oxygen attached to the phosphorus, making them somewhat like esters of phosphonic acids. However, because there is a metal M attached on the other side of the oxygen, they cannot be called normal esters, nor can they be thought of as ester type materials because they do not act like phosphate materials in their reactions with metals and the formation of catalysts. A useful way in which these materials are very like phosphines rather than phosphinic acid esters is that they can still form salts such in a very similar manner to phosphonium salts. In the salt preparations they are reacted with a quaternizing addition compound such as methylmethane sulphonate, methyltrifluoromethanesulfonate, methylbromide, methylbenzenesulfonate or other similar materials form simple addition compounds to form a quaternized phosphonium salt with the appropriate anion such as methanesulfonate, bromide, benzenesulfonate, trifluoromethanesulfonate, etc. Furthermore, such ligands and salts of such ligands do not decompose in the same fashion that other organoxy-phosphorus derivatives. This is because the rearrangements involving oxygen migration allowing the phosphorus/oxygen double bond linkages are essentially blocked by the electron withdrawing nature of the metals on the other side of the oxygen in the case of the present types of ligands and salts. Thus, for all intents and purposes, including naming purposes, it is quite appropriate to consider these materials as pseudo-phosphines, pseudo-arsines, pseudo-phosphonium salts and pseudo arsonium salts. Within these materials, it is preferable to have the atom L be a phosphorus, rather than arsenic.

The preferences expressed with respect to the compound of formula XIX above includes some of the preferred ligands for the complexes of formula XX. Other preferred ligands for these complexes include compounds wherein at least one R group is a fluoro group. In another preferred embodiment, the density of ligand functions on the surface is such that the L atoms are sufficiently close together on the surface to allow ligand functions attached to the surface of the tetravalent metal oxide to function as a chelating ligand, wherein two or more L atoms can attach to a given metal atom or cluster thereof.

Preferred metals for use in the complexes of formula XX include ruthenium, rhodium, iron and platinum. The transition metal clusters as defined above can also be employed. The preference of any of these transition metals is dependent upon the use to which the complex material is to be put. For instance,

for photoreactions using the complexes of formula XX, the transition metal is most preferably ruthenium. A preferred complex of this last class useful for photo reactions is n—$TiO_2$ supported bis(Titania-oxydiphenylphosphine)bis(2,2'-bipyridine)dichloro-ruthenium(II). This is a cationic material and will change valence during the courses of such reactions so for obvious reasons the appropriate anions are necessary to counterbalance the charge. The associated anions can and typically do change even during the course of the reactions because of the change in valence state of the transition metal.

Thus, in one application of the complexes of formula XX, they can be used in a photoreaction process comprising contacting the complex in the presence of water with visible or ultraviolet light to thereby decompose the water. Preferably, such a process is conducted at a pH of between about 5 and about 9.5. The preferred metal is ruthenium complexed by at least two bipyridinic ligands and preferably complexed by a chelating ligand attached to a semiconducting support. The attachment is preferably by way of covalent attachments or linkages which render the metal complex electronically contiguous with the semiconductor electrode. The said semiconductor electrode or semiconductor coated conducting electrode to which the metal complex is linked is preferably connected to a counterelectrode by way of an external circuit through which an electrical load may be applied in order to extract work from the catalyzed photoreaction of water. Alternatively, the obverse situation may be utilized to generate hydrogen, $H_2$, at an electrode by the photoreaction of water either directly or with the application of water wither directly or with the application of a small bias voltage. In any case, the net energy available and usable as hydrogen or electrical energy is considerably larger than without such a photoreaction.

Suitable complexes of formula XX which were prepared include

(127) (Titania-oxydiphenylphosphine)iron(O)tetracarbonyl,

$$[TiO_2/Ti—O—(PH_2)P—Fe(CO)_4];$$

(128) Silica-bis(oxydiphenylphosphine)rhodium(I)acetylacetonate,

$$[SiO_2/Si—O—(Ph_2)P)_2—Rh(I)(C_5H_7O_2)];$$

(129) Titania-bis(oxydiphenylphosphine)tetrarhodium(O)decacarbonyl,

$$[TiO_2/(Ti—O—Ph_2P)_2—Rh_4(O)(CO)_{10}];$$

(130) Titania-bis(oxydiphenylphosphine)triruthenium(O)decacarbonyl,

$$[TiO_2/(Ti—O—(Ph)_2P)_2—Ru_3(CO)_{10}]$$

(131) Titania-bis(oxydiphenylphosphine)dichloroplatinum (II),

$$[TiO_2/Ti—O—(Ph_2)P)_2—cis—PTCl_2];$$

(132) Titania-(oxydiphenylphosphine)carbonylrhodium(I)acetylacetonate,

$$[TiO_2/Ti—O—(Ph)_2P—Rh(CO)(C_5H_2O_2)];$$

(133) Titania-(oxydiphenylphosphine)dicarbonyldichlororuthenium(II),

$$[TiO_2/Ti—O—(Ph)_2P—Ru(CO)_2Cl_2];\text{ and}$$

(134) n-titania-bis(oxydiphenylphosphine)bis(2,2[1]bipyridine)dichlororuthenium(II),

$$[n—TiO_2/Ti—O—(Ph)_2P)_2(H_5NC_5—C_5NH_5)_2RuCl_2].$$

Other suitable complexes within this class are

(134) Thoria-bis(oxy-bis(pentafluorophenyl)phosphine)tricarbonylhydridorhenium(I),

$$[ThO_2/(Th—O—(F_5C_6)_2P)_2—Re(CO)_3H];$$

(135) Zirconia-(oxybis(2-cyanomethyl)phosphine)pentacarbonylmolybdenum(O),

$$[ZrO_2/Zr—O—(NCCH_2CH_2)_2P—Mo(CO)_5];$$

(136) Germanium-(oxydiethylphosphine(copper(I) trifluoromethanesulfonate,

$$[GeO_2/Ge—O—(C_2H_5)_2P—Cu^{\oplus\ominus}O_3SCF_3];$$

(137) Titania-(oxydiferrocenylarsine)tricarbonylnickel(O),

$$[TiO_2/Ti—O—((C_5H_5)Fe(II)((C_5H_4))_2As—Ni(CO)_3];$$

(138) Titania-bis(oxydifluorophosphine)tetradecacarbonylhexarhodium(O),

$$TiO_2/(Ti—O—PF_3)_2Rh_6(CO)_{14}];$$

(139) Titania-bis(oxy(bispentafluorophenyl)fluorophosphine)dicarbonylnickel(O),

$$[TiO_2/(Ti—O—PF(C_6F_5)_2)_2Ni(CO)_2];$$

and other similar materials.

The complexes of formula XX can also be used as catalysts in conversion reactions of hydrocarbons and carbon monoxide, e.g. in reactions such as hydrogenation with hydrogen or CO plus water, dehydrogenation, hydrocarbonylation of olefins, hydrocarbon synthesis, alcohol synthesis, water-gas disproportionation or photo reactions as discussed above, for example, photo reactions of water, including photoelectrolysis of water for the purpose of producing electrical energy or the production of hydrogen, $H_2$.

Still another embodiment of the present invention is directed to a composition of matter (XXI) comprising surface tetravalent metal oxide selected from the group consisting of $SiO_2$, $TiO_2$, $ZrO_2$, $HfO_2$, $ThO_2$, $GeO_2$, $SnO_2$ and mixtures thereof, said metal oxide having a salt group bonded to said metal through an oxide linkage wherein said salt group is a member selected from the formulae

$$—O—{}^{+}L^1R_3 \qquad (XXII)$$

and

$$—O—{}^{+}L^1R_{3-b}[Q(J)_a]_b \qquad (XXIII)$$

Each $L^1$ atom is selected from trivalent P and As. The symbol a is an integer of from 1 to 2, while b is an integer of from 1 to 3. Each R is independently selected from alkyl, aryl, alkoxyalkyl, aryloxyalkyl, alkoxyaryl, aryloxyaryl, ferrocenyl, and fluoro groups, wherein the aryl portions thereof can be substituted with a halogen and the alkyl portions thereof can be substituted with halogen groups, with the proviso that said halogens on said alkyl groups are not in an alpha-position with respect to said L atom unless they are fluoro atoms. The symbol Q is a member selected from the group consisting of alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with halogen groups and wherein said Q group has a+1 points of attachment to L atoms. The symbol J is selected from $LR_2$ and $L^+R_3$, wherein L is independently selected from P and As. The compositions XXI also include if necessary single or multiply charged inorganic or organic, soluble anions having charges sufficient to balance the charges from any $L^+$ moities.

In a preferred embodiment of the compounds of formula XXI the salt group is $—O—{}^{+}L^1R_3$. Another preferred embodiment comprises a compound of XXI wherein the ${}^{+}L^1$ moiety attached to the metal oxide through the oxide linkage contains at least one, more preferably two, and most preferably three aryl groups, preferably phenyl groups. Another preferred group of compounds of formula XXI are those in which a methyl or ethyl group attached to the ${}^{+}L^1$ bonded to the metal oxide through the oxide linkage.

The discussion above with respect to the concentration of L atoms of the metal oxide surface likewise applies here. This concentration is greater than 0.2 of a milliequivalents per gram when the titania has a surface area of 123 square meters per gram. That concentration is barely sufficient for chelation.

The anions associated with these compounds of formula XXI can be essentially any anion balancing the charges of the $L^+$ moieties. Unreactive anions, i.e., anions that are not strongly oxidizing or reducing; are most preferable. In some instances, such as when the materials are to be used as intermediates for subsequent exchange of the anions for another use, it is preferred to have small, relatively polar anions such as chloride, bromide, sulfate, methanesulfonate, $BF_4^-$, $SbF_6^-$ and other similar anions.

The solid materials of the type in which there is an M-O-L type bond attaching the "phosphine" can be quaternized as mentioned previously by reaction with quaternizing addition compounds. Such quaternizing addition compounds cannot be acids; they must be ester or halide materials of the sorts that are used to make conventional quaternized salt compounds. Typical examples of quaternizing addition compounds include methyl methanesulfonate, methyl trifluoromethanesulfonate, methylbenzenesulfonate, methyl bromide, benzyl bromide, benzyl, methanesulfonate, benzyl benzenesulfonate, butyl methanesulfonate, etc. Such quaternizations are accomplished just as if the material being quaternized were a true phosphine rather than the pseudo-phosphine or pseudo-arsine type materials which are actually being quaternized. Thus, in this instance the oxygen moiety on the phosphorus is oxygen and need not be considered different from the substituents in which carbon is attached directly to the phosphorus or arsenic. These quaternized solid materials then become very much equivalent to the other organic and inorganic ion exchange materials in which the solid material has large numbers of quaternary salt functionalities allowing anions to be exchanged at will. The best quaternization agents typically are those

which are very reactive and form very stable quaternary salts. Thus, the methyl and phenyl derivatives are particularly useful in this regard. Other reagents that are particularly useful for quaternizing the pseudo-phosphines and pseudo-arsines include the oxonium and sulfonium salts. Thus, trimethyloxonium and triethyloxonium tetrafluoroborate, triphenyloxonium fluoroborate, trimethylsulphonium and triphenylsulphonium chlorides are all useful in this regard. In fact, the oxonium and sulfonium salts were the reagents of choice used in the preparation of these species of formula XXI.

Suitable compounds of formula XXI, which were synthesized, include

(140) Silica-oxytriphenylphosphonium tetrafluoroborate,

$$[SiO_2/Si—O—P^{\oplus}Ø_3{}^{\ominus}BF_4];$$

(141) Silica-oxydiphenylmethylphosphonium tetrafluoroborate,

$$[SiO_2/Si—O—P^{\oplus}Ø_2(CH_3)^{\ominus}BF_4];$$

(141a) Silica-oxydiphenylethylphosphonium tetrafluoroborate,

$$[SiO_2/Si—O—P^{\oplus}Ø_2(C_2H_5)^{\ominus}BF_4];$$

(142) Titania-oxytriphenylphosphonium tetrafluoroborate,

$$TiO_2/Ti—O—P^{\oplus}Ø_3{}^{\ominus}BF_4];$$

(143) n-Titania-oxytriphenylphosphonium chloride,

$$[n—TiO_2/Ti—O—{}^{\oplus}PØ_3{}^{\ominus}Cl];$$ and

(144) titania-oxydiphenylmethylphosphonium methanesulfonate,

$$[TiO_2/Ti—O—{}^{\oplus}PØ_2(CH_3)CH_3SO_3{}^{-}].$$

Other suitable compounds within this class are

(145) Silica-oxydiphenylmethylphosphonium methanesulfonate

$$[SiO_2/Si—O—P^{\oplus}(CH_3)Ph_2{}^{\ominus}O_3SCH_3];$$

(146) Titania-oxydibenzylphenylarsonium tetrafluoroborate,

$$[TiO_2/Ti—O—As^{\oplus}(CH_2Ph)_2Ph^{\ominus}BF_4];$$

(147) Thoria-bis(oxy-tri-n-butylphosphonium) sulfate,

$$[ThO_2/(Th—O—{}^{\oplus}P(n—C_4H_9)_3)_2SO_4{}^{\ominus}];$$

(148) Silica-oxytrimethylphosphonium triethyl-2-ethylhexylboranate,

$$[SiO_2/Si—O—{}^{\oplus}P(CH_3)_3(C_2H_5)_3B^{\ominus}(CH_2CH(CH_2CH_3)CH_2CH_2CH_2CH_3)];$$

(149) zirconia-oxytriphenylphosphonium hexafluoroantimonate,

$$[ZrO_2/Zr—O—{}^{\oplus}PPh_3{}^{\ominus}SbF_6];$$

(150) n-Titania-oxybenzylidphenylphosphonium bromide,

$$[n—TiO_2/Ti—O—{}^{\ominus}P(Ph_2)(CH_2Ph)Br^{\ominus}];$$

(151) Titania-bis(oxy(bis(2-chloroethyl)phosphonium)-μ-(para-xylylene) bis(tetrabromoaluminate),

$$[TiO_2/(Ti—O—(ClCH_2CH_2)_2P^{\oplus})_2(—CH—p—(C_6H_4)—CH_2—)(^{-}AlBr_4)_2)];$$

and other similar materials.

The compounds of the formula XXI are useful as ion exchange materials for cleaning of liquid streams, recovery of anionic materials from liquid streams, and ion exchangeable supports for exchangeable ligand materials and catalysts made from such materials as discussed below.

The compounds of the formula XXI above are useful in essentially similar, but oppositely changed

roles and uses to the cation exchangeable solid materials discussed above. Thus, these materials can be the preferred choices for many uses in which the cation exchangeable materials are unsuitable or in which less than optimal performance or stability is obtained.

Still another aspect of the present invention involves silicate cluster compositions of matter of the formula

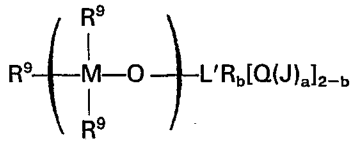 XXIV

In this formula, the symbol a is 0, 1 or 2, while b is 0, 1 or 2. Each L is trivalent P or As. Each R group is independently selected from alkyl, aryl, alkoxyalkyl, aryloxyalkyl, alkoxyaryl, aryloxyaryl, ferrocenyl, and fluorogroups, provided that (1) the aryl portions of said R group can be substituted with a halogen and the alkyl portions of said R group can be substituted with a member selected from halogen and hydroxy groups, (2) said halogens on said alkyl groups are not in an alpha-position with respect to said L atom unless they are fluoro atoms; and (3) the fluoro groups are not attached directly to the M atom. Each Q group is independently selected from alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with a halogen and/or a hydroxy group. The symbol J is selected from $LR_2$ and $L^+R_3$, wherein L is selected from P and A. M is Si, Ti or a mixture thereof. $R^9$ is selected from an R group, an OR group, a group $Q(J)_a$, a group $OQ(J)_a$, and an

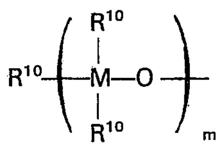

group wherein $R^{10}$ is a member selected from R, OR, $Q(J)_a$ and $OQ(J)_a$, wherein R, Q, J and a are as defined above for formula XXIV, and wherein m is an integer of from 0 to about 200. These compounds also include single or multiply charged inorganic or organic, soluble anions having charges sufficient to balance any charges from $L^+$ moieties contained in the compounds.

In these compounds M preferabiy comprises Si. In one preferred group of compounds within this class M comprises Si and $R^9$ comprises

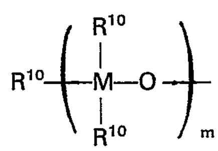

wherein m is an integer of from 1 to 5. Another group of preferred compounds within this class include those in which at least one $R^9$ comprises

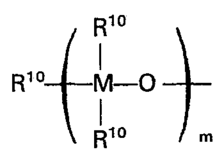

wherein m is independently chosen for each $R^9$ as an integer from 1 to 5. In this last group of compounds, each of the $R^9$ and $R^{10}$ preferably independently are selected from R and/or OR groups. Still another preferred group of compounds within this class are those in which L comprises trivalent P, while another group of preferred compounds are those in which the L group comprises trivalent phosphorus and the J group comprises a quaternized $L^+$ group. In general, the silicate cluster compounds of formula XXIV preferably contain less than 250 non-hydrogen and non-halide atoms, more preferably they contain less than 150 such atoms, and most preferably they contain less than 100 of such atoms.

With the silicate cluster ligands and for that matter to a somewhate lesser extent with all the ligands discussed in the present application, the proper choice of ligands for a desired catalyst and catalyst application includes consideration of the steric bulk of the ligand which might be used. It should be pointed out that the silicone or titanium "cluster" type materials discussed above are among the exceptionally bulky ligands, especially when the total number of non-hydrogen and non-halide atoms is in the upper portion of the range discussed above, and also in instances when m in the range of from 3, 4, 5 and likewise when the various R groups (such as R, $R^1$, $R^2$, $R^9$ etc.) are bulky groups.

The compounds of formula XXIV can be prepared by a novel process in accordance with the present invention. In this novel process an addition reaction is performed between a compound selected from $R_2P{-}PR_2$, $R_2POH$ and $R_2PH$ on the one hand, and a material having M-O-C or M-O-M bonds on the other. The materials containing M-O-M bonds also include the surface tetravalent metal oxides discussed above. This reaction is conducted at a temperature sufficient to cause the desired reaction between said compounds and said material to thereby form the compound of formula XXIV, wherein the R groups are as defined above, provided that the R groups are stable at the reaction temperature. For example, the R groups cannot contain alkyl hydroxyl groups. It should be noted that the compounds of the formulas $R_2P{-}OH$ and $R_2PH$ are in equilibrium with each other, the equilibrium depending upon the temperature of the reaction and the nature of the R substituents. Typically, the temperature of this preparation reaction is in the range of from 200 to 400°C, depending upon the nature of the R substituents and the nature of the substrate material having M-O-C or M-O-M bonds. In this reaction, the preferred M atoms are silicon or titanium, although where the starting material is a solid metal oxide surface, the metals can also be hafnium, zirconium, thorium, germanium, and sometimes tin. The conditions of this reaction also depend upon how thoroughly the solid metal oxide surface is dried and calcined, also upon the surface of the metal oxide and upon the phase or physical structure of the metal oxide reactant, that is, on the extent of surface defects which are the most reactive sites.

Suitable compounds of formula XXIV which have been prepared include

(153) bis(tri-sec-butoxysilicoxy)((diphenylphosphinoxy)di-sec-butoxysilicoxy)methylsilane

$$[((s{-}C_4H_9{-}O)_3SiO)_2Si(CH_3)(O{-}Si(O{-}s{-}C_4H_9)_2(O{-}PPh_2));$$

(154) bis(tri-sec-butoxysilicoxy)(bis(2-cyanoethyl)phosphinoxydi-sec-butoxysilicoxy)methylsilane

$$[((s{-}C_4H_9{-}O)_2Si{-}O)_2Si(CH_3)(O{-}Si(O{-}s{-}C_4H_9)_2(\theta O{-}P(CH_2CH_2CN)_2))];$$

(155) bis(bis(2-cyanoethyl)phosphinoxydi-sec-butoxysilicoxy)(tri-sec-butoxy silicoxy)methylsilane

$$[((NCCH_2CH_2)_2P{-}O{-})(s{-}C_4H_9{-}O{-})_2Si{-}O{-})_2((s{-}C_4H_9{-}O{-})_3Si{-}O){-}SiCH_3];$$ and

(156) (bis (2-cyanoethyl) phosphinoxydi-sec-butoxysilicoxy)((bis)2-cyanoethyl)methyloxyphosphonium) di-sec-butoxysilicoxy(tri- sec-butoxysilicoxy)methylsilane methanesulfonate

$$[((NCCH_2CH_2)_2(CH_3)P^{\oplus}{-}O{-}Si(O{-}S{-}C_4H_9)_2{-}O){-}Si(CH_3)(O{-}Si(O{-}s{-}C_4H_9)_2(O{-}P(CH_2CH_2CH)_2))^{\ominus}O_3SCH_3].$$

Other suitable compounds within this class are

(157) (tributylsilicoxy)diphenylphosphine,

$$[(n{-}C_4H_9)_3Si{-}o{-}PPh_2];$$

(158) (bis (3-(trimethylammonium)butyl)(bis(2-methoxyethyl)silicoxy) (bis(4-hydroxybutyl)phosphine)(4-sulfonato-n-butyl)(tri-n-butyl) boranate,

$$[((CH_3N^{\oplus}CH(CH_3)CH_2CH_2Si(CH_3OCH_2CH_2)O{-}P(CH_2CH_2CH_2CH_2OH)_2{}^{\ominus}O_3SCH_2CH_2CH_2CH_2B^{\ominus}(n{-}C_4H_9)_3];$$

(159) (2-((trimethylsilicoxy) fluoro-phosphino)ethynyl)triphenylarsonium hexafluorosilicate,

$$[((CH_3)_3SiO{-}PF({-}CC{-}As^{\oplus}Ph_3))_2SiF_6{}^{\ominus}];$$

(160) tris(trifluoromethylsilicoxy)phosphine,

$$[((CF_3)_3SiO{-})_3P];$$

(161) tris(tris(2,4,6-trimethylphenoxy)silicoxytitanoxymethylfluorophosphine,

$$[((2,4,6{-}(CH_3)_3(C_6H_2){-}O{-})_3Si{-}O{-})_3Ti{-}O{-}PF(CH_3)];$$

and similar materials.

Another aspect of the present invention comprises the use of the compounds of the formula XXIV as ligands in complexes of formula XXV of Group VIB, VIIB, VIII, or IB transition metals. Again, the metals used in these complexes include the individual transition metals and transition metal clusters as discussed above. The preferred ligands for use in these complexes include the preferred compounds of formula XXIV

as discussed above. Considerable numbers of complexes of formula XXIV have been prepared. Other suitable complexes of formula XXV are

(164) tris((tributylsilicoxy)diphenylphosphine)carbonylhydridorhodium(I),

$$[((n\text{—}C_4H_9)_3Si\text{—}O\text{—}PPh_2)_3Rh(CO)H];$$

(165) tris(((trihexylsilicoxy)-2-ethyl)((dimethyl)silicoxy)diphenylphosphine)carbonylhydridorhodium(I),

$$[((n\text{—}C_6H_{13})_3Si\text{—}O\text{—}Si(CH_3)_2\text{—}O\text{—}PPh_2)Rh(CO)H];$$

(166) tris(((tributylsilicoxy)-2-ethyl)((dibutylsilicoxy)diphenylphosphine)carbonylhydridoiridium(I),

$$[((n\text{—}C_4H_9)_3Si\text{—}O\text{—}Si(n\text{—}C_4H_9)_2\text{—}O\text{—}PPh_2)Ir(CO)H];$$

(167) trimethylsilyloxy)pentadeca(dimethylsilicoxy))diphenylphosphine)tricarbonylnickel(O);

(168) $$[((CH_3)_3Si\text{—}(O\text{—}Si(CH_3))\text{—}O\text{—}PPh_2)Ni(CO)_3];$$

(169) tris((tris(phenoxy)titanium)oxy-bis(ferrocenyl)arsine)nonacarbonyltetrarhodium(O)

$$[((Ph\text{—}O\text{—})_3Ti\text{—}O\text{—}As((C_5H_4)Fe(II)(C_5H_5))_2)_3Rh_4(CO)_9];$$

(170) (((tri-n-butylphosphoniumoxy)(diphenoxy)silicoxy)(di-para-methoxyphenoxy-titanoxy)(diphenoxy-silicoxy)dibutylphosphine) (tri-n-butylphosphine)bis(acetylacetato)ruthenium(II) triethyl-2-ethylhexylboranate,

$$[((n\text{—}C_4H_9)_3P^{\ominus}\text{—}O\text{—}Si(Ph\text{—}O\text{—})_2\text{—}O\text{—}Ti(CH_3O(p\text{—}C_6H_4)\text{—}O\text{—})_2\text{—}O\text{—}Si(Ph\text{—}O\text{—})_2\text{—}O\text{—}P)N\text{—}C_4H_9)_2)$$
$$((n\text{—}C_4H_9)_3P)Ru(C_5H_7O_2)_2(C_2H_5)_3B^{\ominus}(CH_2CH(C_2H_5)CH_2CH_2CH_2CH_3)];$$

(171) tris(((1-((2-(tri-n-butylsilyl)ethyl)(p-tolyl)phosphino)-1'-ferrocenyl)methynyl) (dimethylsilicoxytriethylphosphonium)(dimethyl silicoxy)diphenylphosphine) carboxylhydridorhodium(I) tetrachloroaluminate,

$$[((((n\text{—}C_4H_9)_3SiCH_2CH_2)(p\text{—}CH_3C_6H_4)P)((C_5H_4)Fe(II)(C_5H_4))CH(Si(CH_3)_2O\text{—}^{\oplus}P(C_2H_5)_3)\text{—}$$
$$(Si(CH_3)_2OPPh_2))_3Rh(CO)H^{\ominus}AlCl_4];$$

(172) tris((2-(tri-n-pentylsilyl)ethyl)(ferrocenyl)(para-tolyl)phosphine)carbonylhydridorhodium(I),

$$[(((n\text{—}C_5H_{11})_3SiCH_2CH_2\text{—})((C_5H_5Fe(II)(C_5H_4))(CH_3(p\text{—}C_6H_4))P)_3Rh(CO)H];$$

and similar materials.

The compounds of formula XXIV and the transition metal complexes of formula XXV as described above can be used as solvents and cosolvents, and as catalysts in conversion reactions of hydrocarbons and carbon monoxide, such as hydrogenation with $H_2$ or with carbon monoxide and water, dehydrogenation, hydrocarbonylation of olefins, hydrocarbon synthesis, alcohol synthesis and water-gas disproportionation. Because of the bulkiness of the ligands in the complexes of formula XXV, fewer of the ligands fit around the transition metal atom or cluster thereof which is to be used as a catalyst, thus allowing coordination positions on the transition metal atom or cluster to be available for reactions with substrates and reagents. Alternatively, such bulky ligands arrange themselves on opposite sides of the transition metal atom or cluster thereof due to their large size, thus allowing different coordination positions to be open for reaction with substrate and reagent materials than with less bulky ligands or chelating ligands. Another alternative way in which the bulky ligands of complexes of formula XXV affect the structures of the catalyst materials and therefore the reactions in which they are used is that such bulky ligands, particularly when two or more of such ligands are present on the metal or cluster of metals, often modify the coordination geometry of the ligands on the complex because of the rearrangements of the ligands within the coordination sphere caused by their bulkiness. Examples of this change in coordination geometry include changes from octahedral to tetragonal bipyramidal, square planar to tetragonal square, and pyramidal to trigonal bipyramidal. Bulky ligands such as the compound of formula XXIV would typically occupy the axial positions in trigonal bipyrimidal and tetragonal bipyramidal coordination geometries, whereas less bulky ligands would occupy or could occupy the equatorial positions either solely or as well as the axial positions in such coordination geometries. Likewise, lowest chelating ligands would occupy solely equatorial positions or equatorial positions in combination with axial positions, but in almost no case could it occupy both axial positions.

There are cobalt reaction product catalyst mixtures (XXVII) comprising cobalt metal, ligand, in the presence of $H_2$ and CO at a temperature and pressure sufficient to obtain a catalyst capable of catalyzing a hydrocarbonylation reaction, wherein said ligand comprises a compound of the formula

$$R_{3-b}L'S_b \qquad (XXVI)$$

In the ligand compounds of XXVI, L' is trivalent P or As. Each R is independently selected from alkyl, aryl, alkoxyalkyl, aryloxyalkyl, alkoxyaryl, aryloxyaryl, and ferrocenyl groups, wherein the aryl portions thereof can be substituted with a halogen and the alkyl portions thereof can be substituted with a halogen and or a hydroxy group, with the proviso that said halogens on said alkyl groups are not in an alpha-position with respect to said L atom unless they are fluoro atoms. Each S group is independently selected from F and —C≡Cr$^1$, wherein $R^1$ is R, $L^+R_3$ or $Q^1L^+R_3$, wherein R is defined as above and $Q^1$ is selected from alkyl, alkynyl, aryl, alkoxyalkyl, aryloxyalkyl, alkoxyaryl, aryloxyaryl and ferrocenyl, wherein the aryl portions thereof can be substituted with a halogen and the alkyl portions thereof can be substituted with a halogen and/or a hydroxy group. The symbol b in formula XXVI is an integer of from to 1 to 3.

The ligands of formula XXV as defined above can also be used in another embodiment comprising a complex (XXVIII) containing a transition metal selected from chromium, molybdenum, tungsten, manganese, rhenium, iron, ruthenium, osmium, nickel palladium, platinum, copper, silver and gold and mixtures of such metals along with the ligand of the formula XXVI.

These same ligands of formula XXVI can also be used in another embodiment of the present invention comprising a complex XXIX containing transition metal selected from $Rh^{+1}$ and $Ir^{+1}$ along with at least one ligand having the formula XXVI as defined above. The $Rh^{+1}$ and $Ir^{+1}$ transition metals in these complexes can be present either alone or in combination with rhodium or iridium atoms of other valances or for that matter with other transition metals of varying valencies and other non-transition metals such as in transition metal atom clusters as defined above. In the complexes of formula XXVI, the transition metal preferably comprises $Rh^{+1}$ or Rh in combination with other rhodium atoms in metal clusters containing 4 or 6 rhodium atoms. The tetrarhodium cluster is particularly preferred.

In the complexes of formulae XXVII, XXVIII, and XXIX, the preferred ligands are selected independently from compounds of formula XXVI in which there is only one non-quaternized L atom, compounds of the formula XXVI containing only one non-quaternized L atom and compounds of the formula $R^1_{3-b}LF$ wherein b is an integer of 1, 2 or 3; L is selected from P and As; $R^1$ is selected from R, $L^+R_3$ and $Q^1L^+R_3$, wherein R is selected from alkyl, aryl, alkoxyalkyl, aryloxyalkyl, alkoxyaryl, aryloxyaryl, and ferrocenyl groups, wherein the aryl portions thereof can be substituted with a halogen and the alkyl portions thereof can be substituted with a halogen and/or a hydroxy group, with the proviso that said halogens on said alkyl groups are not in an alpha-position with respect to said L atom unless they are fluoro atoms and with the proviso that at least one R group on said L atom is a fluoro group, and wherein $Q^1$ is selected from alkyl, alkynyl, aryl, alkoxyalkyl, aryloxyalkyl, alkoxyaryl, aryloxyaryl and ferrocenyl, wherein the aryl portions thereof can be substituted with a halogen and the alkyl portions thereof can be substituted with a halogen and/or a hydroxy group.

Suitable complexes of formulae XXVIII and XXIX, which were prepared, include:

(173) bis(bis(ferrocenyl)fluorophosphine)tricarbonylion(O),

$$[(((C_5H_5)Fe(C_5H_4))_2PF)_2Fe(CO)_3];$$

(174) ((2-difluorophosphino)ethynyl)diphenylmethylphosphonium)tetracarbonyliron(O)methanesulfonate,

$$[(\emptyset(CH_3)P^{\oplus}C{\equiv}CPF_2)Fe(CO)_4CH_3SO_3^{\ominus}];$$

(175) ((2-(difluorophosphino)ethynyl)diethylmethylphosphonium)tetracarbonyliron(O)-trifluoromethanesulfonate,

$$[((C_2H_5)_2(CH_3)P^{\oplus}C{\equiv}CPF_2)Fe(CO)_4(F_3CSO_3)^{\ominus}];$$

(176) bis(pentafluorophenyldifluorophosphine)dicarbonylnickel(O),

$$[((C_6F_5)PF_2)_2Ni(CO)_2]$$

(177) tris(di-tertiary-butylfluorophosphine)tricarbonylmolybdenum,

$$[(((CH_3)_3C)_2PF)_3Mo(CO)_3]; \text{ and}$$

(178) tris(pentaphenylethynyldifluorophosphine)nonacarbonyl triruthenium(O),

$$[(C_6F_5C{\equiv}C{-}PF_2)_3Ru_3(CO)_9].$$

The preparation of all the salt ligands within the present invention were done by techniques well-known in the art, with minor modifications to suit the particular solubilities, volatilities and stoichiometries involved. Specifically, the salts were made by contacting the "ligand" material to be quaternized with a quaternizing addition compound such as methylmethansulfonate, dimethylsulfate, methyl fluoroborate, methylbenzenesulfonate, trimethyloxoniumfluoroburate, triphenyloxy-oniumfluoroburate, triethyloxoniumfluoroborate, triphenylsulfoniumchloride, octylbromide, 2-ethylhexylbromide, methyliodide and other similar sorts of compounds with the appropriate structure for the desired added materials to form the quaternary phosphoniumarsonium, etc. compound. Such materials were added under conditions of very high dilution reaction which is a well-known art and evolving somewhat over the past century. The quaternizing addition compound was added in appropriate stoichiometric amount to turn part or a fraction of the ligand moieties of an otherwise chelating ligand into a partial salt ligand compound. This means that, for example, one of two phosphine functionalities in an otherwise diphosphine chelating ligand is turned into a phosphonium salt leaving the other one present as the free phosphine still usable as a ligand moiety.

Most of the quaternizing addition compounds and most of the chelating ligands which were starting materials to form the salt ligands were obtained directly from commercial sources. Others were made by very standard techniques from precursors which were available. In such a situation, small amounts of non-salt chelating ligands were present in the reaction product mixture. These di-salt non-ligand materials are present due to the inevitable statistical distributions except that the reactions being run in very high dilution and with such other precautions helped to minimize the undesired reaction products. Normal recrystallization techniques were followed wherein typically between four and six recrystallizations were sufficient to purify the salt ligand compounds. The elemental analyses and other analyses showed these materials and others to be pure enough within experimental error to be usable for the formation of pure metal complexes for use as catalysts. Other proofs of structure which were obtained on selected example amidst the wide variety of salt ligand materials formed were acid titrations of the amount of remaining ligand and infrared data confirmation of structures. Very detailed information was obtained on the purity of the materials by such means, and whether or not the functional groups were present, etc. In all cases in which materials were made, they were purified and accepted as the desired materials only after elemental analysis, for all the elements obtainable, e.g., oxygen is not available, were within experimental error of the calculated theoretical composition.

A typical reaction to produce a partial salt ligand, is the reaction of bis(diphenylphosphino)ethane or diphos in an 0.25 mole quantity dissolved in 2.5 liters of purified toluene in a 5 liter flask in which the solvent was refluxing at approximately 112° to 115°C under an $N_2$ atmosphere. High-speed stirring was used An 0.25 mole quantity of methyl methanesulfonate was dissolved in 1 liter of purified toluene and the solution was added drop-wise over six days at the rate of one drop per 5 seconds, with an extra 2½ days of refluxing after the final addition to make sure that the reaction was complete. The work-up of the material which typically coated the sides of the flask was quite standard and was done in a manner to prevent contamination by $H_2O$, air, etc. After dissolution and recrystallization four times, the reaction gave 0.20 moles of the product (2-(diphenylphosphino)ethyl)methyldiphenylphosphonium methanesulfonate.

Obviously, this preparation is a typical simple addition reaction run at a high dilution to form the quaternary salt. This type of reaction was basically a production-scale run on a high dilution system. This type of reaction was done many many times to make the various partial salt materials used as salt ligands herein. The preparations of the non-ligand salt solvents were even easier and still very conventional. They involved the complete quaternization of the ligand functionalities by the use of the stoichiometric plus ten percent amounts of quaternizing addition reagents. The starting ligands were typically only single ligands, although a few chelating materials were converted to polyquaternary salts. The other difference in the reactions was that high dilution conditions were not necessary and the apparatus was thus much simpler.

The fluorophosphine ligands typically were made, likewise, by a conventional substitution reaction in which fluoride was substituted for chloride on the ligand material or precursor. Chlorine-containing materials, that is, dichlorophosphines and chloroorganic phosphines were obtained commercially or were made from readily available starting materials in conventional manner. Substitution of fluorine for chlorine again was a typical substitution reaction in which the chloro-organophosphine was passed across a fluorine salt, potassium-fluorosulfite, a common fluorine exchange medium, at high temperatures, typically between 100° and 200°C., depending on the volatility of the compound in question and its reactivity. The fluorine product materials were much more volatile and were removed by distillation from the insoluble and non-volatile potassium chloride. The volatile $SO_2$ byproduct was easily separable from the organofluorophosphine during distillation. Distillation and recrystallization were used for purifications depending upon the volatility of the materials. Elemental analyses again were used as primary proof of structure, in combination with sequential reactions and elemental analyses of the subsequent reaction products as well.

The acetylenic phosphines were made by simply taking the sodium acetylide salts formed by reaction of commercially available acetylenes with sodamide with byproduct ammonia eliminated. The sodium acetylide was then reacted with the phosphorus halides to make the appropriate phosphine precursors which was then used to make the quaternized salt in the manner described above. These materials as well, like all of the others, were submitted for elemental analysis after purification for proof of structure.

Elemental analysis on all available elements, especially metals phosphorus, carbon, hydrogen, nitrogen, arsenic, were performed on all of the metal complexes and agreed with an experimental error with the calculated theoretical elemental compositions. Consequently, since the materials were simply substitution reactions and very little could happen which was unexpected in such simple substitution reactions where a ligand is substituted for, in general, carbon monoxide. This was considered quite sufficient proof of structure for the metal complexes. In addition, on a selected number of materials, infrared analysis were used to confirm many portions of the structures which were susceptible to Ir or infrared analytical techniques. In particular, carbonyl stretching frequencies and similar such peaks were confirmed in the appropriate regions for a significant number of the metal complexes which were synthesized. The results of the many tests of hydrogenation using hydrogen as a reagent were uniformally excellent and essentially complete during the running of the wide variety of metal complexes. A selected number of some 25 approximate metal catalyst systems were tested for their hydrogenation capabilities in the test of hydrogenation of cyclohexene with the metal catalyst chosen from all of the major groupings of catalyst synthesized herein, and the results were uniformly good with hydrogenation at the standard conditions at room temperature and 50 PSig hydrogen pressure in dimethylether for 1 hour. In all cases, 97 to 100% cyclohexane product as shown by the GC. Typically 3 cycles were run in each test in order to provide evidence that the catalyst was not being irreversably changed during the course of such reaction.

Dehydrogenation reactions were typically run on the same catalyst as the hydrogenation reactions and the conditions shown above were the dehydrogenation tests. In all cases, benzene formed at 50 pounds pressure of the inert gas nitrogen, simply by contacting the 1,4-cyclohexadiene with catalyst quantities of about .01 to .02 mmoles per 5 grams of substrate cyclohexadienes. In the poorest case, the amount of benzene yield was over 98%. Likewise, 3 cycles typically showed the same thing and even in those cases where it was as low as 98.93% in the first cycle, it got better to the point where after on the third cycle it was 100.0%. Thus, it is fairly obvious that this broad range of catalysts all have very good dehydrogenation capability, just as they all have broadly hydrogenation capability.

The formation of the metal complexes from the metal salts or other metal complexes is also quite straight-forward and well-known in the art. Simple exchange reactions, particularly when a volatile ligand is involved, such as carbon monoxide, which it almost is in this sort of reaction since the starting material metal complexes are 0 valent or low valent metal salts in almost all circumstances. This means that simply the stoichiometrically desired amount of ligand or ligands is added to the mixture in a solvent which is typically toluene, ethyl alcohol, tetrahydrofuran, dimethyl ether or mixtures thereof. The materials were heated until the carbon monoxide was driven off and the material reached equilibrium, typically done at solvent reflux or slightly above, or if the solvent, like dimethylether boils very low, the reaction is done in an autoclave and heated up to 80° to 100°C. The other solvents typically reflux between 60° and 110°C. Reactions are very mild for these conversions. In some cases, in order to preserve the identity of the catalyst or convert to the appropriate material, synthesis gas in 1:1 ratio, that is, hydrogen and carbon monoxide in 1:1 ratio was typically applied as an atmosphere at approximately $1.72\times10^6$ N/m$^2$ to $2.07\times10^6$ N/m$^2$ (250 to 300 psig) in order to help convert the appropriate materials to their carbonylhydrides. Syngas pressure was applied in essentially all the cases with cobalt, rhodium, and iridium during the formation of the catalysts. The other metals did not need such application, and such complex preparations were made in an inert atmosphere $N_2$ rather than under syngas atmosphere.

A catalyst which was useful in hydrocarbonylation, hydrogenation, dehydrogenation, alcohol and hydrocarbonsynthesis and water-gas shift was made using $Rh_2(CH_3CO_2)_4$ as the rhodium starting material and the ligands (2-(diphenylphosphino)ethyl)methyldiphenylphosphonium methanesulfonate. Stoichiometric amounts of 3 equivalents of the ligand with 1 equivalent of rhodium were used to make the product which had a general formula of $L_3Rh(CO)H$. In this case, 13.6 mmoles of the ligand were reacted with 2.3 mmoles of the dirhodium specie or 4.55 mmoles of rhodium metal with a result that after recrystallization three times to purify the product, 3.4 mmoles of product was obtained. The reaction was run under carbon monoxide $H_2$ syngas pressure of 304 psig with syngas in 1:1 ratio with THF solvent under nitrogen in a Teflon-lined autoclave at 80°C. for 17 hours. ('Teflon' is a registered Trade Mark) After distilling off the solvent, all the work-up and recrystallizations were also done under nitrogen. This typical method is known in the art for the preparation of this type of metal complex. Likewise, the other metal complexes in the present invention were prepared by similar well-known techniques in the art with minor modifications depending on the metals and the ligands.

Hydrogenation tests in which $H_2$ hydrogen was the reducing agent were done in the Teflon-lined autoclave at $3.4\times10^5$ N/m$^2$ (50 psig) hydrogen pressure using cyclohexene as the substrate. The solvent was 200 milliliters of dimethyl ether and 5.0 grams of cyclohexene feed was treated in the autoclave using 0.1 millimoles of catalyst, either in slurry form if the catalyst was a solid or in solution if it were a soluble catalyst. The reaction was run at 25°C ambient temperature and with a time per cycle of one hour. At the end of a cycle the ether and the ether solvent and the hydrocarbon product and starting materials were distilled off; the ether fractionated away from the resulting materials which were then injected into a GC and analyzed for cyclohexane percentage relative to cyclohexene starting material. The catalyst remained in the autoclave reactor vessel to which was then added an additional aliquot of solvent and an additional aliquot of feed material the cyclohexene for another cycle. Typically three cycles were run in each test in

order to determine whether the catalyst was being irreversably changed during the course of such reactions. In a small proportion of cases the solvent was changed as noted.

The dehydrogenation test cases were run in a manner very similar to the hydrogenation test cases. However, the feed material was 1,4-cyclohexadiene and the gas above the solution was nitrogen $N_2$ pressurized to 50 psig in order to keep the low boiling solvent dimethylether as a liquid. One hour cycles were again used and 5 gram aliquots of feed as well. The reactions were run at 25°C and the pressure within the reactor was allowed to increase as it would with the evolution of the hydrogen. Work-up of the reaction products was identical and the GC results were used to determine percentage of benzene relative to other cyclohexane, cyclohexene, cyclohexadiene products. Again the amount of catalyst used was in the .01 to .02 millimole range and typically three cycles were run in order to determine whether any irreversable damage or changes were occurring to the catalysts or catalyst mixture. In every case most of the reaction for the dehydrogenation evidently occured almost immediately upon addition of the feed substrate material to the reaction mixture. The reaction was so fast that it could be watched in terms of the rise of pressure immediately upon addition of the cyclohexadiene. This despite the fact that the reaction was basically endothermic and a decrease in temperature was occurring simultaneously with the production of hydrogen.

The testing for the reduction using carbon monoxide and water was typically done in a completely different mode. Specifically again, it was done in this 500 cc capacity Teflon®-lined autoclave, but if the solvent was then 1 to 1 by volume, dimethyl ether to water, 200 milliliters total, reaction was done at 80°C and under a CO pressure of $2.5\times10^6$ N/m$^2$ (355 psig). Again, the same quantity of catalyst was used; same catalyst range of .01 to .02 millimoles of catalyst and 5 gram charges of cyclohexene were used as the substrate. Ths cycle lengths were $2\frac{1}{2}$ hrs. long unless otherwise noted. In these reactions typically only part of the cyclohexene was reduced, somewhere around 50% under the reaction conditions and in addition a certain amount of cyclohexane aldehyde was formed as an oxynation product. Typically the oxynation product was in the 2 or 3% range. Such catalysts, because there was water present and so forth, were also tested for hydrogenation using $H_2$ for comparison and were also checked for water gas shift reaction and dehydrogenation also.

The conditions under which the cobalt oxynation reactions or cobalt hydrocarbonylation reactions were run are considerably modified from the conditions under which actual commercial units run, partially due to the limitations of the apparatus and partially due to the fact that the reactions were run in a Teflon-lined autoclave which had certain constraints on pressure and temperature. Thus, the hydrocarbonylation reactions were run under much milder conditions than the hydrocarbonylation reactions are run in normal commercial cobalt catalyzed systems. Because the reactions were run under very mild conditions which with standard cobalt catalysts results in no significant reaction, they indicate that our novel phosphine ligands do result in a comparative improvement in the overall process. Furthermore because these reactions were run under such mild conditions where very little of the starting material olefin was reacted the overall differences in normal to iso aldehydes yields also are indicative of what would happen under the much more severe conditions under which commercial units would run. Thus, it should be noted that the results reported herein are all internally consistent and comparable to one another. Those materials which work better at lower conditions would also work better at the higher conditions but are that much better and more useful and desirable in that respect also. Thus, when hydrocarbonylation tests were done using no additional phosphine, essentially no reaction was accomplished over the course of the normal run, and after one hour at 225°C only 4/10% of the propylene had reacted. Still at 225°C the addition of triphenylphosphine gave essentially the same results, whereas lowering the temperature to 165°C and adding tributyl phosphine allowed the reaction to go to the level of .85% of the propylene being reacted in the same time span. With phenyldifluorophosphine as the addition material, 9.4% of the propylene starting material reacted and with diphenylfluorophosphine, 5.08% of the propylene reacted; and finally with the difluorophosphinodiethylmethylphosphonium trifluoromethanesulfonate phosphine being used, 10.11% of the propylene had reacted in one run at 165°C and 23.63% of the propylene had reacted in another cycle at 200°C. Thus, quite clearly, the more of the fluorines or pseudo-fluorines that are on the phosphine that is used as the ligand, the better the catalyst in terms of the rate of catalysis. Thus, these materials make the reaction go at less severe conditions. Furthermore, the improvement in normal to iso ratio of product butraldehydes improves in the same direction with a similar correlation: the more fluoro or pseudo-fluoro substituents on the phosphine, the higher the normal to iso ratio.

The testing for water gas shift typically was done in a similar sort of system to that for testing with CO/water reduction of olefins where some gas was pressured into the reactor at approx. $2.5\times10^6$ N/m$^2$ (355 psig) at 80°C using a solvent in a 1 to 1 ratio of 200 milliliters split between dimethylether and water. Again, 0.1 to 0.2 millimoles of catalyst were used and the cycle lengths were not of concern because samples of gas were taken in a flow through system after specific times. The reactions were done at atmospheric pressure and 100°C as well at the temperature of boiling water and samples were taken at specific times for GC analysis and mass spec analysis. Typical times taken for samples were at the start of the reaction essentially 0 minutes, generally approximately 30 minutes time and at an hour and two hours and an overnight sample, typically on the order of 16 to 17 hours after the start of the run in order to determine whether there was any catalyst degradation during the period of time or improvement.

The testing for hydrogenation of olefins using water and carbon monoxide was done on relatively few catalysts because of the demonstrable ubiquity of hydrogenation capability as demonstrated by the hydrogenation catalysis results and dehydrogenation catalysis results as reported above. In a typical reaction, .05 mmoles catalysts which was, for example, a bis ligand platinum dichloride. The ligand was tris((diethylmethylphosphonium)ethynylphosphine)trismethanesulfonate which obviously was changed during the course of the reaction, because the resulting catalyst which was removed from the reactor showed, by Ir analysis, a platinum-carbonyl bonding. The reaction was done in a dimethylether-water solvent 1:1 by volume of 200ml of salt-solvent at 80°C. in the Teflon-lined autoclave with stirring, and with 2½ hour cycles. The catalyst was dissolved in the solvent and work-up was by distillation of the solvent and product from the catalyst and analysis tests by GC. In the first cycle of such a test, there was 49.2% cyclohexane and 46.8% remaining starting material cyclohexene, with 4% other materials primarily cyclohexanealdehyde. The second cycle yielded 54.9% cyclohexane with correspondingly lower amounts of the other two materials, and the third cycle produced 55.1% cyclohexane with correspondingly lower amounts of the other materials. So obviously the catalyst was improving in its efficiency over the course of the reaction for the reduction of cyclohexene to cyclohexane. Its activity was quite evident even though the reaction was considerably slower than a hydrogenation using hydrogen itself. The infrared analysis of the catalyst showed that the carbon carbon triple bonds still existed after such a reaction so that the platinum was not hydrogenating its own ligands. At higher temperature in the fourth cycle at 120°C. 92.2% cyclohexane was obtained.

A large number of tests were made for testing hydroformylation of olefins, particularly of propene with the improved yields of the desired normal butraldehyde and minimizing the amount of iso butyraldehyde. Likewise, the goal of running such hydroformylation reactions at less severe conditions, that is at lower temperatures and pressures and higher feed rates of syngas and propylene feed materials was also realized. Advantage was taken of the zero volatility of the salt ligands and of the resulting complexes and salt solvents. A continuous hydroformylation reaction process was demonstrated in outline, at least, because all products were distilled from the reaction mixture solvent-catalyst mixture before analysis. This test is typical of a continuous reaction run batchwise, the only way possible in this laboratory to approximate continuous commercial processing. The catalyst and solvent systems stayed *in situ* unless the light ethers were used as solvents. In such hydrocarbonylation reactions run as described above, the typical results of the hydroformylation tests were comparable. Thus, a representative test was made using the catalyst tris(2-(diethylmethylphosphonium) ethyl)(diethylphosphino)(methylsulfonate) rhodium-hydridocarbonyl in diethyl ether solvent.

The first test at 80°C. in a 2 hours cycle time showed 29% of the starting material remaining, and gave 89.3% of product materials of which there was normal butraldehyde and 7.2% iso-butraldehyde with a ratio of about 12.4. Some 4/10% of propane was observed in the product and 3.5% non-butraldehyde of which a 2.7% portion was methanol and 0.3% was ethyleneglycol. In the next cycle, a 1 hour cycle, as 125°C. In which only 3/10% of starting material remained, of the products 85% was normal and 9.5% isobutyaldehydes for a ratio of 9.0 with about ½% propane and some 5.3% non-butraldehydes of which the greatest proportion or 4.6% was methanol and about a tenth of a percent ethyleneglycol was present. In the third cycle at 150°C. for ½ hour, all the starting material was reacted and of the products, about 81% was normal and 13% isobutraldehydes for a ratio of about 6:1 and about 6.2% was non-butraldehydes of which 8/10 of a percent was propane and 4.9% was methanol and a tenth of a percent ethyleneglycol was present again. Cycle 4 at 155%C. this time shifting from the diethylether solvent to THF as a solvent again for ½ hour showed all of the starting material reacted and the products were 81.2% normal and 13.4% isobutraldehydes. There were 5.4% non-butraldehydes of which 0.6% was propane and 2.7% methanol and 7/10 of a percent $C_6$ materials. A considerable excess of the ligand was then added to make the ratio of about 32:1. The reaction again was run in THF at 155°C. so that the THF and the excess ligand were the co-solvents again for ½ hour. The product materials were 92% normal and 3.5% isobutraldehyddes for an n:i ratio of 25.3 which was a considerable improvement. There were 4.4% non-butraldehydes of which about a 2.9% portion was methanol and 2/10 of a percent was propane. Methanol was still produced even though THF was the ether-type solvent or co-solvent. A duplication of the previous cycle showed 9.24% normal and 3.6% isobutraldehydes for a ratio of 25.4. There were 4.0% non-butraldehydes of which about 0.1% was propane and 3.1% methanol. Cycle 7 then was run with no propylene feed but the other conditions in this and there was 24.2% methanol produced, along with 3/10 of a percent ethylene glycol, traces of propanetriol, 0.7% methane, and 0.9% $C_2$ to $C_8$ paraffins. Thus, the aldohol and hydrocarbon production using this catalyst was demonstrated. Cycle 8 was the same, but where an additional equivalent amount of water was added and no ether was present as solvent. The salt-ligand and water were co-solvents. The product was 32.7% methanol, 0.2% ethylene glycol, and the gas going through in a continuous process showed 70.6% hydrogen, 8.7% CO, 20.7% $CO_2$, as well as about 0.4% dimethylcarbonate. Thus, this cycle shows both water-gas disproportionation catalysis as well as further optimization of alcohol synthesis and the Fischer-Tropsch catalysis was somewhat less. Cycle 9 was done again hydrocarbonylation as in Cycles 5 and 6. The product was 92.6% normal and 3.45% isobutraldehydes and 4.0% non-butraldehydes, 0.15% starting material propylene, 2/10 of a percent propane and 2.7% methanol. An additional equal amount of ligand was added further before Cycle 10 and the reactions run again. The product was 93.5% normal and 3.15% isobutraldehydes for a ratio of 29.7 and 3.5%

non-butraldehydes of which 1.4% was methanol. Cycle 11 involved a further aloquot addition of ligands to give 94.7% n and 2.6% isobutraldehydes for a ratio of 36.4 n:i and Cycle 12 with a similarly added yet another aloquot to give 94.5% n and 2.45% isobutraldehyde for a ratio of 38.0% n:i. Cycle 13 was again run with no propylene feed under the same conditions with the salt solvent as the only solvent but with the syngas feed to give 36.1% methanol, 5.5% ethylene glycol and 4/10 of a percent propanetriol as well as traces of butanetetrol and pentalrythritol. A duplicate cycle 14 using longer feed times, 80°C. for 24 hours instead of the higher temperature of 150°C. for the shorter period of time, gave 36.3% methanol, 8.45 ethyleneglycol, and 2/10 of a percent higher glycols. Thus, alcohol synthesis, hydrocarbonsynthesis, and very favorable normal to isobutraldehyde ratios from hydrocarbonylation are demonstrable with this particular catalyst. Other catalysts gave similar sorts of results depending on the ligands, the conditions and the metals involved, but the overall ligands, solvents, catalysts, and processes worked to give favorable results.

The compositions and processes of the present invention can be used in catalytic conversion of hydrocarbons and carbon monoxide in the reactions, hydrogenation, dehydrogenation, hydrocarbonylation of olefins, hydrocarbon synthesis, alcohol synthesis and water-gas disproportionation.

**Claims**

1. A composition comprising a compound of the formula:

$$R_{4-b}L^{+}[QC{\equiv}C{-}(-J)]_{c}[{-}Q{-}(-J)_{a}]d \qquad \text{I}$$

or

$$R_{3-b'}L[C{\equiv}CQ{-}(-J)_{a'}]_{c'}[{-}Q{-}(-J)_{a'}]_{d'} \qquad \text{II}$$

wherein a and a' are 0, 1 or 2; b is an integer of from 1 to 4; b' is 1, 2 or 3; c+d equals b, c' and d' equals b'; c is an integer of from 1 to 4; c' is an integer of from 1 to 3; d is 0, 1, 2 or 3; and d' is 0, 1 or 2; each L is a trivalent P or As atom; each R group is independently selected from alkyl, aryl, alkoxyalkyl, aryloxyalkyl, alkoxyaryl, aryloxyaryl, ferrocenyl, and fluoro groups, wherein the aryl portions thereof can be substituted with a halogen and the alkyl portions thereof can be substituted with a member selected from halogen and hydroxy groups, provided that said halogens on said alkyl groups are not in an alpha-position with respect to said L atom unless they are fluoro atoms; each Q group is independently selected from a covalent bond, alkyl, alkynyl, aryl, alkoxyalkyl, aryloxyalkyl, alkoxyaryl, aryloxyaryl and ferrocenyl, wherein the aryl portions thereof can be substituted with a halogen and the alkyl portions thereof can be substituted with halogen and/or hydroxy groups; wherein said Q groups have a+1 points of attachment to L atoms; J is selected from $LR_2$ and $L^+R_3$ with the proviso that at least one R group on the L atom or atoms (but not $L^+$ atoms) is a fluoro group, said composition including if necessary single or multiply charged inorganic or organic, non-solid anions having charges sufficient to balance the charges from any $L^+$ moieties on the compound.

2. A complex comprising a Group VIB, VIIB, VIII, or IB transition metal in complex association with a ligand, said ligand comprising surface tetravalent metal oxide selected from $SiO_2$, $TiO_2$, $ZrO_2$, $HfO_2$, $ThO_2$, $GeO_2$, $SnO_2$ and mixtures thereof and attached to the surface of said tetravalent metal oxide a coordinating group of the formula $-L^1R_2$ or $-L^1R_{2-b}{-}[{-}Q{-}(-J)_a]_b$, wherein each $L^1$ atom is trivalent P or As; a is 1 or 2; b is 1 or 2; each R is independently selected from alkyl, aryl, alkoxyalkyl, aryloxyalkyl, alkoxyaryl, aryloxyaryl, ferrocenyl, and fluoro groups, wherein the aryl portions thereof can be substituted with a halogen and the alkyl portions thereof can be substituted with halogen groups, with the proviso that said halogens on said alkyl groups are not in an alpha position with respect to said L atom unless they are fluoro atoms; Q is alkyl, alkynyl, aryl, alkoxyalkyl, aryloxyalkyl, alkoxyaryl, aryloxyaryl, or ferrocenyl, wherein the aryl portions thereof can be substituted with a halogen and the alkyl portions thereof can be substituted with halogen groups, and wherein said Q group has a+1 points of attachment to L atoms; J is $LR_2$, or $L^+R_3$, wherein L is independently selected from P and As; and said coordinating group is attached to said tetravalent metal oxide through an oxide linkage.

3. A complex comprising a group IB, VIB, VIIB or VIII transition metal and an ion exchanged ligand composition comprising (1) a solid cationic support comprising surface tetravalent metal oxide selected from $SiO_2$, $TiO_2$, $ZrO_2$, $HfO_2$, $ThO_2$, $GeO_2$, $SnO_2$ and mixtures thereof, and attached to the surface of said tetravalent metal oxide, a salt group of the formula $^{\pm}L^1R_3$ or $^{\pm}L^1R_{3-b}{-}[{-}Q{-}(-J)_a]_b$, wherein each $L^1$ atom is trivalent P or As; a is an integer of from 1 to 2; b is an integer of from 1 to 3; each R is independently selected from alkyl, aryl, alkoxyalkyl, aryloxyalkyl, alkoxy aryl, aryloxyaryl, ferrocenyl, and fluoro groups, wherein the aryl portions thereof can be substituted with a halogen and the alkyl portions thereof can be substituted with halogen and/or hydroxy groups, with the proviso that said halogens on said alkyl groups are not in an alpha position with respect to said L atom unless they are fluoro atoms; Q is a member selected from alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with halogen and/or hydroxy groups and wherein said Q group has a+1 points of attachment to L atoms; J is selected from $LR_2$ and $L^+R_3$ and wherein each L is independently P or As; said composition also including if necessary single or multiply charged inorganic or organic, soluble anions having charges sufficient to

balance the charges from any $L^+$ moieties, wherein said salt group is attached to said tetravalent metal oxide through an oxygen linkage; and (2) an anion of the formula $R^1_3B^-QLR^1_2$ wherein each $R^1$ group independently represents a member selected from an R group wherein L, Q and R are as defined above.

4. A catalytic conversion process comprising contacting a reactant comprising hydrocarbons and a mixture of (1) carbon monoxide and (2) hydrogen, water or a mixture thereof with a conversion catalyst in a reaction zone maintained at conversion conditions, wherein said catalyst comprises the complex according to claim 3.

5. A complex comprising a Group VIB, VIIB, VIII or IB transition metal and a ligand of the formula:

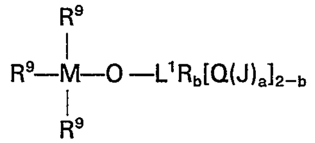

wherein a is 0, 1 or 2; b is 0, 1 or 2; each $L^1$ is trivalent P or As; each R group is independently selected from alkyl, aryl, alkoxyalkyl, aryloxylalkyl, alkoxyaryl, aryloxyaryl, ferrocenyl and fluoro groups, provided that (1) the aryl portions of said R group can be substituted with a halogen and the alkyl portions of said R group can be substituted with a member selected from halogen and hydroxy groups, (2) said halogens on said alkyl groups are not in an alpha position with respect to said L atom unless they are fluoro atoms; and (3) the fluoro groups are not attached directly to the M atom; each Q group is independently selected from alkyl, alkynyl, aryl, alkoxyalkyl, aryloxyalkyl, alkoxyaryl, aryloxyaryl and ferrocenyl, wherein the aryl portions thereof can be substituted with a halogen and the alkyl portions thereof can be substituted with a member selected from halogen and hydroxy groups, J is selected from $LR_2$ and $L^+R_3$, and wherein each L is independently P or As; M is Si, Ti or mixtures thereof; $R^9$ is selected from an R group, an OR group, a group $Q(J)_a$, a group $OQ(J)_{a'}$ and an

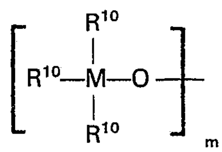

group wherein $R^{10}$ is a member selected from R, OR, $Q(J)_a$ and $OQ(J)_{a'}$ wherein R, Q, J and a are as defined above, and wherein m is 0 or an integer of up to 200; said ligand including if necessary single or multiply charged inorganic or organic, non-solid anions having charges sufficient to balance any charges from $L^+$ moieties on said ligand.

6. A complex comprising a transition metal selected from rhodium and iridium, and mixtures thereof, provided that said rhodium and iridium are in their +1 valence states, and a ligand having the formula $R_{3-b}L\$_b$ wherein L is trivalent P or As; each R is independently selected from alkyl, aryl, alkoxyalkyl, aryloxyalkyl, alkoxyaryl, aryloxyaryl, and ferrocenyl groups, wherein the aryl portions thereof can be substituted with a halogen and the alkyl portions thereof can be substituted with a halogen and/or a hydroxy group, with the proviso that said halogens on said alkyl groups are not in an alpha position with respect to said L atom unless they are fluoro atoms; each \$ group is independently selected from F and $-C{\equiv}CR^1$, wherein $R^1$ is R, $L^+R_3$ or $Q^1L^+R_3$, wherein R is defined as above and $Q^1$ is selected from alkyl, alkynyl, aryl, alkoxyalkyl, aryloxyalkyl, alkoxyaryl, aryloxyaryl, and ferrocenyl, wherein the aryl portions thereof can be substituted with a halogen and the alkyl portions thereof can be substituted with a halogen and/or a hydroxy group; and b is an integer of from 1 to 3 and with the proviso that at least one R group on the L atom (but not $L^+$ atom) is a fluoro group.

7. A complex comprising a Group IB, VIB, VIIB or VIII transition metal and a ligand of the formula:

$$R_{4-b}L^+{+}Q{+}J)_a]_b \qquad \text{XVII}$$

$$R_{3-b'}L{+}Q{+}J)_{a'}]_{b'} \qquad \text{XVIII}$$

wherein L, J, a, a', b and b' are the same as defined in claim 1; each R group is independently selected from alkyl, aryl, alkoxyalkyl, aryloxyalkyl, alkoxyaryl, aryloxyaryl, ferrocenyl, and fluoro groups, wherein the aryl portions thereof can be substituted with a halogen and the alkyl portions thereof can be substituted with a member selected from halogen and hydroxy groups, provided that said halogens on said alkyl groups are not in an alpha-position with respect to said L atom unless they are fluoro atoms; and wherein when a is zero each Q group is independently selected from alkyl, alkynyl, aryl, alkoxyalkyl, aryloxyalkyl, alkoxyaryl, aryloxyaryl and ferrocenyl, wherein the aryl portions thereof can be substituted with a halogen and the alkyl portions thereof can be substituted with halogen and/or hydroxy groups; wherein said Q groups have a+1 points of attachment to L atoms; and wherein when a is not zero Q is a corresponding divalent radical having a+1 points of attachment to L atoms; and with the provisos (1) that in said ligands of the formulae

XVII and XVIII, J contains at least one L atom which is a quaternarised $L^+$ and at least one other L atom which is a non-quaternarised L, (2) that the complex contains a carbonyl group or an acetylaceto group and (3) that when Q is alkylene at least R attached to $L^+$ must be aryl.

**Patentansprüche**

1. Zusammensetzung gekennzeichnet durch eine Verbindung mit der Formel

$$R_{4-b}L^+[QC{\equiv}C(J)]_c[-Q(J)_a]_d \qquad I$$

oder

$$R_{3-b'}L[C{\equiv}CQ(J)_{a'}]_{c'}[-Q(J)_{a'}]_{d'} \qquad II$$

in der a und a' 0, 1 oder 2 sind, b eine ganze Zahl von 1 bis 4 ist, b' 1, 2 oder 3 ist, c+d gleich b ist, c'+d' gleich b' ist, c eine ganze Zahl von 1 bis 4 ist, c' eine ganze Zahl von 1 bis 3 ist, d 0, 2 oder 3 ist, d' 0, 1 oder 2 ist, jedes L ein trivalentes P- oder As-Atom ist, jede R-Gruppe unabhängig voneinander ausgewählt ist aus Alkyl-, Aryl-, Alkoxyalkyl-, Aryloxyalkyl-, Alkoxyaryl-, Aryloxyaryl-, Ferrocenyl- und Fluorgruppen, wobei die Arylreste mit einem Halogen und die Alkylreste mit Halogen- oder Hydroxygruppen substituiert sein können, vorausgesetzt, daß die Halogene an den Alkylgruppen sich nicht in einer α-Stellung zum L-Atom befinden, es sei denn, es handelt sich um Fluoratome, jede O-Gruppe unabhängig ausgewählt ist aus Alkyl-, Alkinyl-, Aryl-, Alkoxyalkyl-, Aryloxyalkyl-, Alkoxyaryl-, Aryloxyaryl- und Ferrocenylgruppen, wobei die Arylreste mit einem Halogen und die Alkylreste mit Halogen- und/oder Hydroxygruppen substituiert sein können und die Q-Gruppen a+1 Verbindungspunkte zu den L-Atomen haben, und J ausgewählt ist aus $LR_2$ und $L^+R_3$, wobei die Maßgabe gilt, daß mindestens eine R-Gruppe an dem L-Atom oder den L-Atomen (aber nicht an den $L^+$-Atomen) eine Fluorgruppe ist, und, wenn erforderlich, durch einfach- oder mehrfach geladene anorganische oder organische, nicht feste Anionen mit Ladungen, die ausreichen, die Ladungen jeglicher $L^+$-Gruppen in der verbindung auszugleichen.

2. Komplex gekennzeichnet durch ein Gruppe VIB, VIIB, VIII oder IB Übergangsmetall in komplexer Verbindung mit einem Liganden, der oberflächlich vierwertiges Metalloxid ausgewählt aus $SiO_2$, $TiO_2$, $ZrO_2$, $HfO_2$, $ThO_2$, $GeO_2$, $SnO_2$ und Mischungen derselben und eine an die Oberfläche des vierwertigen Metalloxids gebundene Koordinationsgruppe mit der Formel $-L^1R_2$ oder $-L^1R_{2-b}[Q(J)_a]_b$ umfaßt, in de jedes $L^1$-Atom ein dreiwertiges P- oder As-Atom ist, a 1 oder 2 ist, b 1 oder 2 ist, jede R-Gruppe unabhängig ausgewählt ist aus Alkyl-, Aryl-, Alkoxyalkyl-, Aryloxyalkyl-, Alkoxyaryl-, Aryloxyaryl-, Ferrocenyl- und Fluorgruppen, wobei die Arylreste mit einem Halogen und die Alkylreste mit Halogengruppen substituiert sein können, mit der Maßgabe, daß die Halogene an den Alkylgruppen sich nicht in α-Stellung zum L-Atom befinden, as sei denn es sind Fluoratome, Q Alkyl, Alkinyl, Aryl, Alkoxyalkyl, Aryloxyalkyl, Alkoxyaryl, Aryloxyaryl oder Ferrocenyl ist, wobei die Arylreste mit einem Halogen und die Alkylreste mit Halogengruppen substituiert sein können une wobei die Q-Gruppe a+1 Verbindungspunkte zu L-Atomen hat, J $LR_2$ oder $L^+R_3$ ist, wobei L unabhängig ausgewählt ist aus P und As, wobei die Koordinationsgruppe an das vierwertige Metalloxid über eine Oxidbindung gebunden ist.

3. Komplex gekennzeichnet durch ein Gruppe IB, VIB, VIIB oder VIII Übergangsmetall und eine ionenausgetauschte Ligandenzusammensetzung, die (1) einen festen kationischen Träger, der oberflächliches vierwertiges Metalloxid ausgewählt aus $SiO_2$, $TiO_2$, $ZrO_2$, $HfO_2$, $ThO_2$, $GeO_2$, $SnO_2$ und Mischungen derselben und gebunden an die Oberfläche des vierwertigen Metalloxids eine Salzgruppe der Formel $^{\pm}L^1R_3$ oder $^{\pm}L^1R_{3-b}[Q(J)_a]_b$ umfaßt, in der jedes $L^1$-Atom ein dreiwertiges P- oder As-Atom ist, a eine ganze Zahl von 1 bis 2 ist, b eine ganze Zahl von 1 bis 3 ist, jedes R unabhängig ausgewählt ist aus Alkyl-, Aryl-, Alkoxyalkyl-, Aryloxyalkyl-, Alkoxyaryl-, Aryloxyaryl-, Ferrocenyl und Fluorgruppen, wobei die Arylreste mit einem Halogen und die Alkylreste mit Halogen- und/oder Hydroxygruppen substituiert sein können, mit der Maßgabe, daß die Halogene an den Alkylgruppen sich nicht in α-Stellung zum L-Atom befinden, es sein denn, sie sind Fluoratome, Q ausgewählt ist aus Alkyl, Alkinyl, Aryl, Alkoxyalkyl, Aryloxyalkyl, Alkoxyaryl, Aryloxyaryl und Ferrocenyl, wobei die Arylreste mit einem Halogen und die Alkylreste mit Halogen oder Hydroxygruppen substituiert sein können und wobei die Q-Gruppe a+1 Verbindungspunkte zu L-Atomen aufweist, und J ausgewählt ist aus $LR_2$ und $L^+R_3$, wobei L unabhängig P oder As ist, wobei die Zusammensetzung, wenn erforderlich, einfach oder mehrfach geladenen anorganische oder organische, lösliche Anionen mit ausreichender Ladung, um die Ladung der $L^+$-Gruppen auszugleichen, enthält und wobei die Salzgruppe an das vierwertige Metalloxid über eine Sauerstoffbindung gebunden ist, und (2) ein Anion der Formel $R^1_3B^-QLR_2^1$ umfaßt, wobei jede $R^1$-Gruppe unabhängig eine R-Gruppe bedeutet und wobei L, Q und R die oben angegebene Bedeutung besitzen.

4. Katalytisches Umwandlungsverfahren, bei dem ein Kohlenwasserstoffe enthaltender Reaktand und eine Mischung aus (1) Kohlenmonoxid und (2) Wasserstoff, Wasser oder einer Mischung derselben mit einem Umwandlungskatalysator in einer auf Umwandlungsbedingungen gehaltenen Reaktionszone kontaktiert werden, wobei der Katalysator durch den Komplex gemäß Anspruch 3 gekennzeichnet ist.

5. Komplex gekennzeichnet durch ein Gruppe VIB, VIIB, VIII oder IB-Übergangsmetall und einen Liganden der Formel:

$$\begin{array}{l} \qquad\quad R^9 \\ \qquad\quad | \\ R^9—M—O—L^1R_b[Q(J)_a]_{2-b} \\ \qquad\quad | \\ \qquad\quad R^9 \end{array}$$

in der a 0, 1 oder 2 ist, b 0, 1 oder 2 ist, jedes $L^1$ dreiwertiges P oder As ist, jede R-Gruppe unabhängig ausgewählt ist aus Alkyl-, Aryl-, Alkoxyalkyl-, Aryloxyalkyl-, Alkoxyaryl-, Aryloxyaryl-, Ferrocenyl- und Fluorgruppen, vorausgesetzt, daß (1) die Arylreste der R-Gruppe mit einem Halogen und die Alkylreste der R-Gruppe mit Halogen- oder Hydroxygruppen substituiert sein können und (2) die Halogene an den Alkylgruppen sich nicht in α-Stellung zu dem L-Atom befinden, es sei denn, sie sind Fluoratome, und (3) die Fluorgruppen nicht direkt an das M-Atom gebunden sind, jede Q-Gruppe unabhängig ausgewählt ist aus Alkyl, Alkinyl, Aryl, Alkoxyalkyl, Aryloxyalkyl, Alkoxyaryl, Aryloxyaryl und Ferrocenyl, wobei die Arylreste mit einem Halogen und die Alkylreste mit Halogen- oder Hydroxygruppen substituiert sein können, J ausgewählt ist aus $LR_2$ und $L^+R_3$, wobei jedes L unabhängig P oder As ist, M Si, Ti oder Mischungen derselben ist, $R^9$ ausgewählt ist aus einer R-Gruppe, einer OR-Gruppe, einer Gruppe $Q(J)_a$, einer Gruppe $OQ(J)_{a'}$ und einer Gruppe mit der Formel

$$\left[ \begin{array}{c} R^{10} \\ | \\ R^{10}—M—O— \\ | \\ R^{10} \end{array} \right]_m$$

in der $R^{10}$ ausgewählt ist aus R, OR, $Q(J)_a$ und $OQ(J)_{a'}$, wobei R, Q, R und a die oben angegebene Bedeutung besitzen, und in der m 0 oder eine ganze Zahl von bis zu 200 ist, wobei der Ligand ebenfalls, wenn erforderlich, einfach oder mehrfach geladene anorganische oder organische, nicht feste Anionen mit für den Ausgleich beliebiger Ladungen von $L^+$-Gruppen in der Zusammensetzung ausreichender Ladung enthält.

6. Komplex gekennzeichnet durch ein Übergangsmetall ausgewählt aus Rhodium und Iridium und Mischungen derselben, vorausgesetzt, daß das Rhodium und das Iridium sich im +1-Valenz-zustand befinden, und einen Liganden mit der Formel $R_{3-b}LS_b$, in der L trivalentes P oder As ist, jedes R unabhängig ausgewählt ist aus Alkyl-, Aryl-, Alkoxyalkyl-, Aryloxyalkyl-, Alkoxyaryl-, Aryloxyaryl- und Ferrocenylgruppen, wobei die Arylreste mit einem Halogen und die Alkylreste mit einem Halogen und/oder eine Hydroxygruppe substituiert sein können, mit der Maßgabe, daß die Halogene an den Alkylgruppen sich nicht in α-Stellung zum dem L-Atom befinden, es sein denn sie sind Fluoratome, jede S-Gruppe unabhängig ausgewählt ist aus F und $—C{\equiv}CR^1$, wobei $R^1$ R, $L^+R_3$ oder $Q^1L^+R_3$ ist, wobei R die oben angegebene Bedeutung hat und $Q^1$ ausgewählt ist aus Alkyl, Alkinyl, Aryl, Alkoxyalkyl, Aryloxyalkyl, Alkoxyaryl, Aryloxyaryl und Ferrocenyl, wobei die Arylreste mit einem Halogen und die Alkylreste mit einem Halogen und/oder eine Hydroxygruppe substituiert sein können, und b eine ganze Zahl von 1 bis 3 ist, mit der Maßgabe, daß mindestens eine R-Gruppe am L-Atom (aber nicht am $L^+$-atom) eine Fluorgruppe ist.

7. Komplex gekennzeichnet durch ein Gruppe IB, VIB, VIIB oder VIII-Übergangsmetall und einen Liganden der Formeln:

$$R_{4-b}L^+\!\!-\!\![Q\!-\!(J)_a]_b \qquad \text{XVII}$$

$$R_{3-b'}L\!-\![Q\!-\!(J)_{a'}]_{b'} \qquad \text{XVIII}$$

in denen L, J a, a', b und b' dieselbe Bedeutung wie in Anspruch 1 haben, jede R-Gruppe unabhängig ausgewählt ist aus Alkyl-, Aryl-, Alkoxyalkyl-, Aryloxyalkyl-, Alkoxyaryl-, Aryloxyaryl-, Ferrocenyl- und Fluorgruppen, wobei die Arylreste mit einem Halogen und die Alkylreste mit Halogen- oder Hydroxygruppen substituiert sein können, vorausgesetzt, daß die Halogene an den Alkylgruppen sich nicht in α-Stellung zu dem L-Atom befinden, es sei denn, sie sind Fluoratome, und, wenn a 0 ist, jede Q-Gruppe unabhängig ausgewählt ist aus Alkyl, Alkinyl, Aryl, Alkoxyalkyl, Aryloxyalkyl, Alkoxyaryl, Aryloxyaryl und Ferrocenyl, wobei die Arylreste mit einem Halogen und die Alkylreste mit Halogen- und/oder Hydroxygruppen substituiert sein können und die Q-Gruppen a+1 Verbindungspunkte zu den L-Atomen aufweisen, und, wenn a nicht 0 ist, Q ein entsprechender zweiwertiger Rest mit a+1 Verbindungspunkten zu den L-Atomen ist, mit den Maßgaben, daß (1) in den Liganden der Formeln XVII und XVIII J mindestens ein L-Atom, das ein quaternisiertes $L^+$ ist, und mindestens ein anderes L-Atom, das ein nicht quaternisiertes L ist, enthält (2) der Komplex eine Carbonylgruppe oder eine Acetylacetogruppe enthält und (3), wenn Q Alkylen ist, zumindest an $L^+$ gebundenes R Aryl sein muß.

5. Complexe comprenant un métal de transition du groupe VIB, VIIB, VIII ou IB, et un ligand de formule:

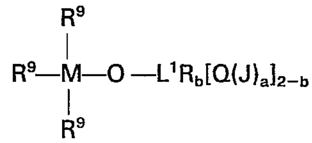

où a vaut 0, 1 ou 2; b vaut 0, 1 ou 2; chaque $L^1$ est P ou As trivalent; chaque groupe R est choisi indépendamment parmi les groupes alkyle, aryle, alcoxyalkyle, aryloxyalkyle, alcoxyaryle, aryloxyaryle, ferrocényle et fluoro, à la condition que (1) les fragments aryle dudit groupe R puissent être substitués par un halogène et les fragments alkyle dudit groupe R puissent être substitués par un radical choisi parmi les groupes halogènes et hydroxy, (2) lesdits halogènes se touvant sur ces groupes alkyle ne soient pas en position alpha par rapport à l'atome L à moins qu'il ne s'agisse d'atomes de fluor; et (3) les groupes fluoro ne soient pas fixés directement à l'atome M; chaque groupe Q est indépendamment choisi parmi les groupes alkyle, alcynyle, aryle, alcoxyalkyle, aryloxyalkyle, alcoxyaryle, aryloxyaryle et ferrocényle, où leurs fragments aryle peuvent être substitués par un halogène et leurs fragments alkyle peuvent être substitués par un radical choisi parmi les groupes halogènes et hydroxy, J est choisi parmi $LR_2$ et $L^+R_3$, et où chaque L est indépendamment P ou As; M est Si, Ti ou leurs mélanges; $R^9$ est choisi parmi un groupe R, un groupe OR, un groupe $Q(J)_a$, un groupe $OQ(J)_a$, et un groupe

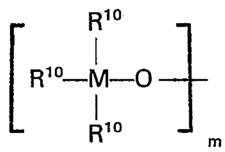

où $R^{10}$ est un radical choisi parmi R, OR, $Q(J)_a$ et $OQ(J)_a$, où R, Q, J et a sont définis comme ci-dessus, et où m vaut 0 ou est un entier pouvant aller jusqu'à 200; ledit ligand renfermant, si nécessaire, des anions, non-solides, organiques ou minéraux, à charge unique ou multiple, possédant des charges suffisantes pour équilibrer toutes charges des fragments $L^+$ se trouvant sur ledit ligand.

6. Complexe comprenant un métal de transition choisi parmi le rhodium et l'iridium, et leurs mélanges, du moment que lesdits rhodium et iridium se trouvent dans leurs état de valence +1, et un ligand possédant la formule $R_{3-b}LS_b$, où L est P ou As trivalent; chaque R est indépendamment choisi parmi les groupes alkyle, aryle, alcoxyalkyle, aryloxyalkyle, alcoxyaryle, aryloxyaryle et ferrocényle, où leurs fragments aryle peuvent être substitués par un halogène et leurs fragments alkyle peuvent être substitués par un groupe halogène et/ou hydroxy, à la condition que lesdits halogènes sur ces groupes, alkyle ne soient pas en position alpha par rapport à l'atome L à moins qu'il ne s'agisse d'atomes de fluor; chaque groupe S étant indépendamment choisi parmi F et $-C{\equiv}R^1$, où $R^1$ est R, $L^+R_3$ ou $Q^1L^+R_3$, où R est défini comme ci-dessus et $Q^1$ est choisi parmi les groupes alkyle, alcynyle, aryle, alcoxyalkyle, aryloxyalkyle, alcoxyaryle, aryloxyaryle et ferrocényle, où leurs fragments aryle peuvent être substitués par un halogène et leurs fragments alkyle peuvent être substitués par un groupe halogène et/ou hydroxy; et b est un entier de 1 à 3 et à la condition que au moins un groupe R se trouvant sur l'atome L (mais non sur l'atome $L^+$), soit un groupe fluoro.

7. Complexe comprenant un métal de transition du groupe IB, VIB, VIIB ou VIII, et un ligand de formule

$$R_{4-b}L^+[-Q-(J)_a]_b \qquad \text{XVII}$$

$$R_{3-b'}L-[Q-(J)_{a'}]_{b'} \qquad \text{XVIII}$$

où L, J, a, a', b et b' sont définis comme dans la revendication 1; chaque groupe R est indépendamment choisi parmi les groupes alkyle, aryle, alcoxyalkyle, aryloxyalkyle, alcoxyaryle, aryloxyaryle, ferrocényle et fluoro, où leurs fragments aryle peuvent être substitués par un halogène et leurs fragments alkyle peuvent être substitués par un radical choisi parmi les groupes halogène et hydroxy, à la condition que lesdits halogènes se trouvant sur lesdits groupes alkyle ne soient pas en position alpha par rapport à l'atome L sauf qu'il s'git d'atomes de fluor; et où, quand a vaut 0, chaque groupe Q est indépendamment choisi parmi les groupes alkyle, alcynyle, aryle, alcoxyalkyle, aryloxyalkyle, alcoxyaryle, aryloxyaryle et ferrocényle, où leurs fragments aryle peuvent être substitués par un halogène et leurs fragments alkyle peuvent être substitués par des groupes halogène et/ou hydroxy; où les groupes Q possèdent a+1 points de fixation aux atomes L: et où, quand a est différent de 0, Q est un radical bivalent correspondant possédant a+1 points de fixation aux atomes L; et à la condition (1) que, dans les ligands de formules XVII et XVIII, J contienne au moins un atome L; qui est un $L^+$ quaternarisé et au moins un autre atome L qui est un L non-quaternarisé, (2) que le complexe contienne un groupe carbonyle ou un groupe acétylacéto, et (3) que, quand Q est le radical alkylène, au moins un R fixé à $L^+$ soit le groupe aryle.

**Revendications**

1. Composition comprenant un composé de formule:

$$R_{4-b}L^{+}[QC\equiv C\text{-}(\text{-}J)]_{c}[-Q\text{-}(\text{-}J)_{a}]_{d} \quad \text{I}$$

or

$$R_{3-b'}L[C\equiv CQ\text{-}(\text{-}J)_{a'}]_{c'}[-Q\text{-}(\text{-}J)_{a'}]_{d'} \quad \text{II}$$

où a et a′ valent 0, 1 ou 2; b est un entier de 1 à 4; b′ vaut 1, 2 ou 3; c+d égale b, c′ et d′ égale b′; c est un entier de 1 à 4; c′ est un entier de 1 à 3; d vaut 0, 1, 2 ou 3; et d′ vaut 0, 1 ou 2; chaque L est un atome de P ou As trivalent; chaque groupe R est, indépendamment des autres, choisi parmi les radicaux alkyle, aryle, alcoxyalkyle, aryloxyalkyle, alcoxyaryle, aryloxyaryle, ferrocényle et fluoro, où les fragments aryle de ces radicaux peuvent être substitués par un halogène et leurs fragments alkyle peuvent être substitués par un radical choisi parmi les groupes halogènes et hydroxy, à la condition que dans ces groupes alkyle, lesdits halogènes ne soient pas dans une position alpha par rapport à l'atome L à moins qu'il ne s'agisse d'atomes de fluor; chaque groupe Q est, indépendamment des autres, choisi parmi une liaison covalente, un groupe alkyle, alcynyle, aryle, alkoxyalkyle, aryloxyalkyle, alcoxyaryle, aryloxyaryle et ferrocényle, où les fragments aryle de ces groupes peuvent être substitués par un halogène et leurs fragments alkyle peuvent être substitués par des groupes halogènes et/ou hydroxy; où les groupes Q possèdent a+1 points de fixation aux atomes L; J est choisi parmi $LR_2$ et $L^+R_3$ avec la condition que au moins un groupe R sur l'atome ou les atomes L (mais non les atomes $L^+$), soit un groupe fluoro, ladite composition renfermant si nécissaire des anions non-solides, minéraux ou organiques, à charge simple ou multiple, possédant suffisamment de charges pour équilibrer les charges provenant de tout fragment $L^+$ se trouvant sur le composé.

2. Complexes comprenant un métal de transition des groupes VIB, VIIB, VIII ou IB, en association complexe avec un ligand, ce ligand comprenant un oxyde superficiel de métal tétravalent choisi parmi $SiO_2$, $TiO_2$, $ZrO_2$, $HfO_2$, $ThO_2$, $GeO_2$, $SnO_2$ et leurs mélanges et, fixé à la surface de cet oxyde de métal tétravalent, un groupe de coordination de formule $-LR_2$ ou $-L^1R_{2-b}\text{-}[\text{-}Q\text{-}(\text{-}J)_a]_b$ où chaque atome $L^1$ est P ou As trivalent; a vaut 1 ou 2; b vaut 1 ou 2; chaque R est, indépendamment des autres, choisi parmi les groupes alkyle, aryle, alcoxyalkyle, aryloxyalkyle, alcoxyaryle, aryloxyaryle, ferrocényle et fluoro, où leurs fragments aryle peuvent être substitués par un halogène et leurs fragments alkyle peuvent être substitués par des groupes halogènes, à la condition que lesdits halogènes se trouvant sur ces groupes alkyle ne soient pas en position alpha par rapport audit atome L sauf s'il s'agit d'atomes de fluor; Q est un groupe alkyle, alcynyle, aryle, alcoxyalkyle, aryloxyalkyle, alcoxyaryle, aryloxyaryle et ferrocényle, où leurs fragments aryles peuvent être substitués par un halogène et leurs fragments alkyle peuvent être substitués par des groupes halogènes, et où le groupe Q possède a+1 points de fixation aux atomes L; J est $LR_2$ ou $L^+R_3$, où L, indépendamment, est choisi parmi P et As; et ledit groupe de coordination est fixé audit oxyde de métal tétravalent par une liaison oxyde.

3. Complexe comprenant un métal de transition du groupe IB, VIB, VIIB ou VIII et une composition de ligand ayant subi un échange d'ions, comprenant (1) un support cationique solide comprenant un oxyde de métal tétravalent en surface choisi parmi $SiO_2$, $TiO_2$, $ZrO_2$, $HfO_2$, $ThO_2$, $GeO_2$, $SnO_2$ et leurs mélanges et, fixé à la surface de l'oxyde de métal tétravalent, un groupe sel de formule $^{\pm}L^1R_3$ ou $^{\pm}L^1R_{3-b}[-Q\text{-}(\text{-}J)_a]_b$, où chaque atome $L^1$ est P ou As trivalent; a est un entier de 1 à 2; best un entier de 1 à 3; chaque R, indépendamment, est choisi parmi les groupes alkyle, aryle, alcoxyalkyle, aryloxyalkyle, alcoxyaryle, aryloxyaryle, ferrocényle et fluoro, où leurs fragments aryle peuvent être substitués par un halogène et leurs fragments alkyle peuvent être substitués par des groupes halogènes et/ou hydroxy, à la condition que les halogènes se trouvant sur les groupes alkyle ne soient pas en position alpha par rapport à l'atome L sauf s'il s'agit d'atomes de fluor; Q est un groupe choisi parmi les radicaux alkyle, alcynyle, aryle, alcoxyalkyle, aryloxyalkyle, alcoxyaryle, aryloxyaryle ou ferrocényle, où leurs fragments aryle peuvent être substitués par un halogène et leurs fragments alkyle peuvent être substitués par des groupes halogènes et/ou hydroxy, et où le groupe Q possède a+1 points de fixation aux atomes L; J est choisi parmi $LR_2$ et $L^+R_3$, et où chaque L est indépendamment P ou As; ladite composition renfermant également, si nécessaire, des anions solubles, minéraux ou organiques, à charge unique ou multiple, possédant suffisamment de charges pour équilibrer les charges provenant de tout fragment $L^+$, où ledit groupe sel est fixé audit oxyde métal tétravalent par une liaison oxygène; et (2) un anion de formule $R^1_3B^-QLR^1_2$ où chaque groupe $R^1$ représente indépendamment un radical choisi parmi un groupe R, où L, Q et R sont définis comme ci-dessus.

4. Procédé de conversion catalytique consistant à mettre en contact un réactif contenant des hydrocarbures et un mélange (1) de monoxyde de carbone et (2) d'hydrogène d'eau ou un mélange de ces derniers, avec un catalyseur de conversion dans une zone de réaction maintenue dans les conditions de conversion, ledit catalyseur contenant le complexe selon la revendication 3.